# EUROPEAN PATENT SPECIFICATION

(11) **EP 3 061 013 B1**
(45) Date of publication and mention of the grant of the patent: **22.04.2020**
(21) Application number: 14789282.2
(22) Date of filing: 25.10.2014
(51) Int. Cl.: G16H 20/10, G16H 10/60

(54) **PATIENT CARE SYSTEM REPORTING ADHERENCE TO TREATMENT REGIMEN**
PATIENTENBEHANDLUNGSSYSTEM ZUM MELDEN DER EINHALTUNG EINES BEHANDLUNGSREGIMES
SYSTÈME DE SOINS AUX PATIENTS REPORTANT L'ADHÉSION AU RÉGIME THÉRAPEUTIQUE

(30) Priority: 25.10.2013 EP 13190396; 05.12.2013 EP 13195960
(43) Date of publication of application: 31.08.2016
(73) Proprietor: Ares Trading SA, 1170 Aubonne (CH)
(72) Inventor: LAKE, Colin, Boston, Massachusetts 02111 (US); PATERSON, Andrew, Bridge of Allan Stirling Central Scotland FK9 4AU (GB); EXELL, Simon, 1202 Geneva (CH); CHANIE, Eric, CH-1207 Geneva (CH); KOUVAS, Georgios, CH-1202 Geneva (CH)
(74) Representative: Merck Serono S.A. Intellectual Property
(86) International application number: PCT/EP2014/072922
(87) International publication number: WO 2015/059306

(56) References cited:
- US-A1- 2003 221 687
- US-A1- 2007 016 443
- ARTUR ROCHA ET AL: "ICT4Depression: Service oriented architecture applied to the treatment of depression", COMPUTER-BASED MEDICAL SYSTEMS (CBMS), 2012 25TH INTERNATIONAL SYMPOSIUM ON, IEEE, 20 June 2012 (2012-06-20), pages 1-6, XP032227009, DOI: 10.1109/CBMS.2012.6266379 ISBN: 978-1-4673-2049-8
- "iMed TM. Electronic Multiple Sclerosis Patient Clinical Database. User Manual. Version: 6.", , 1 October 2012 (2012-10-01), pages 1-45, XP055142068, Retrieved from the Internet: URL:http://www.imed.org/merck_serono_imed/ en/images/iMed%20User%20Manual_tcm383_1523 9.pdf [retrieved on 2014-09-23]

## Description

### Field of the invention

The present invention relates to an electronic system to monitor and assist in patient therapy and wellness, in particular patients suffering a chronic condition, such conditions including neurodegenerative diseases, particularly multiple sclerosis, and growth hormone deficiency. Other diseases which may also benefit from the present invention are rheumatoid arthritis, juvenile rheumatoid arthritis, psoriasis, plaque psoriasis, Crohn's disease, juvenile Crohn's disease, asthma, psoriatic arthritis, ulcerative colitis, systemic lupus erythematosus and ankylosing spondylitis.

Monitoring and assisting in patient therapy and wellness includes, in one of the specific aspects of the invention, monitoring the efficacy of a treatment regimen and in particular the efficacy of a dosage regimen.

### Background

It is known to use an electronic injection device to administer a pre-determined dosing regimen. For example, WO 2005/077441, WO 2006/085175, WO 2006/085204 or WO 2007/088444 disclose an electronic injection device marketed under the registered trademarks RebiSmart and Easypod. Patient adherence to a pre-determined dosing regimen is often lower than 100%. This is because a patient may forget to inject or they may inject at the wrong time. Furthermore, a patient may intentionally omit injections due to pain or side effects of the medicament.

One limitation of known electronic injection devices is that it is not possible for the devices to measure the efficacy of the dosage regimen followed by the patient using the device. As such, the patient must recall from memory the extent to which they adhered to their pre-determined dosing regimen and report this information to a physician. The patient must also recall from memory the extent to which their dosing regimen affected their physiological state and report this information to a physician. The physician must then piece this information together to determine the extent to which the patient adhered to their pre-determined dosing regimen. The physician must also piece this information together to determine the extent to which adherence to the pre-determined dosing regimen influenced the patient's physiological state. It is possible that the patient may not be able to sufficiently recall or record their adherence to a pre-determined dosing regimen and the affect that their adherence had on their physiological state. This is particularly problematic in the case where a patient suffers from a neurodegenerative disordered, such as multiple sclerosis, where their memory may be affected. This problem is confounded by the fact that the patient's appointments with their physician may be spaced apart by prolonged periods of time, such as up to 6 months. Furthermore, the patient's appointments with the physician may be very short, e.g. less than 15 minutes long. As such, there may not be enough time during an appointment for a physician to accurately determine the efficacy of a treatment regimen. Thus, there is a need in the art for a system to determine the efficacy of a treatment regimen, such as a dosage regimen.

Patients suffering from chronic conditions, for instance neurodegenerative diseases, usually meet their physician or other health care professionals (HCP) at regular intervals and may receive assistance and services from field nurses or other members of patient support organisations. As mentioned above the intervals may be spaced apart by prolonged periods of time that are not optimal to monitor the patient's condition, take corrective measures, provide optimal treatment, or provide accompanying therapeutic and wellness services as a function of the state of the patient's condition.

Many of the before mentioned diseases have a severe impact on the health-related quality of life, with somatic symptoms having a direct influence on emotional and social functioning. For instance, it is common for patients with chronic diseases to fall into a depression and experience relational problems. Additionally, the health-related quality of life may vary from individual to individual, depending on the level of somatic problems, personality traits, capability to mentally handle the disease and capability to maintain relationships and a social life. Compounding the issue of the negative impact of diseases such as Multiple Sclerosis on the health-related quality of life are the challenges faced by health care practitioners in managing patients due to high patient heterogeneity with regard to disease subtype, severity, comorbidities, symptoms, the impact of symptoms on the health-related quality of life, and the short-lived nature of symptoms. Often, standard clinical assessments do not provide enough information for HCPs to effectively manage the disease and improve health-related quality of life.

US2007/016443 discloses a medication compliance system and medical dosage device for treating chronic diseases such as hypertension, hypercholesterolemia and osteoporosis. The system and device in US2007/016443 aim to improve a patient's adherence to a dosage regime of a prescribed medication. The medical dosage device is provided with sensors to determine if and when a dose of medicament has been taken. A feedback system provides feedback information to the patient on the adherence to the prescribed dosage regime and a messaging system creates tailored messages to the patient through the most appropriate communication channel.

US2003/221687 discloses a system for treating depression which comprises different modules related to medicament treatment, cognitive measures and exercise therapy. However, neither US2007/016443 nor US2003/221687 are adapted to assess the correlation between a multitude of parameters defining a patient's physiological state and a medication regimen.

There is also a need for many diseases to gain a better understanding of the effects of the treatment regime on the health of the patient, this being difficult for diseases, for instance Multiple Sclerosis and Growth Hormone deficiency, where the effects of drug administration have a large temporal offset from the time of administration. In these cases tracking the efficacy of a particular treatment regime is particularly difficult.

### Summary of the invention

An object of the invention is to provide a patient care system that improves patient treatment monitoring for health care professionals and that improves patient wellness and care.

It is advantageous to provide a patient care system that provides reliable, relevant and easily readable information for decision making for health care professionals (HCP).

It is advantageous to provide a patient care system that facilitates ease of administering medications for patients.

It is advantageous to provide a patient care system that provides easy health communication between HCPs and patients.

It is advantageous to provide a patient care system to provide accompanying therapeutic and wellness services as a function of the state of the patient's condition.

It is advantageous to provide a patient care system that enables reliable and economical tracking of the efficacy of a treatment regime, especially for diseases, such as Multiple Sclerosis and Growth Hormone deficiency, where the effects of drug administration have a large temporal offset from the time of administration.

The present invention provides a patient care system according to independent claim 1.

Various advantageous features of the invention are set forth in the dependent claims.

Disclosed herein is a patient care system comprising a medical device for administering a medical treatment to a patient and a server system configured to receive and transmit data via a communications network to, respectively from users including patients and health care professionals, the server system further configured to process and store data related to patient care. The server system comprises a database configured to encrypt and store encrypted data related to patient care, an application server including patient care software components for disease management and patient information management program, a communication server including a web server software application for data transfer through the internet, the patient care software components operable to receive medical device usage data comprising data on the usage of said medical device transferred through the communications network, and further operable to process said medical device usage data in conjunction with patient data to generate a report or a plurality of reports related to the treatment of the patient, the reports being accessible remotely via the communications network by registered users of the patient care system as a function of respective roles and privileges of the registered user stored in the server system.

The patient information management program comprises a reports component configured to generate reports, in the form of tables, charts, lists, diagrams or graphical representations based on information selected from drug administration history, adherence data, patient outcome reports, patient health reports, patient physiological data reports, medical device settings, treatment regimen data, and any combination of aforesaid information, and the reports component is configured to form composite reports for simultaneous display on a user interface device (UID) display, including composite adherence and patient outcome reports to facilitate evaluation of the effects of non-adherence to the treatment regimen or the efficacy of the medical treatment.

The present invention provides a holistic care system for assessing the efficacy of treatment and health-related quality of life, which enables health care professionals (HCPs) to improve medication and therapy in a patient's treatment regimen.

Another advantage of the present patient care system is that it not only enables HCPs, but also makes it possible for patients to self-monitor their treatments and the associated somatic and psychological effects, whereby the patients are more likely to become active in disease management and treatment decision-making, which in turn may lead to improved treatment compliance, and ultimately, improved outcomes.

A further advantage is that since the application does not limit patients with regards to the number of instruments completed or frequency of reporting, HCPs may configure the system to schedule certain PRO assessments at certain time intervals (e.g. monthly) for their interests. HCPs may therefore use the system to actively monitor patients.

The communication server may advantageously further comprise a remote service data upload software application configured for wireless telecommunication technology (WTT) data transfer using a cell phone network, to allow users with mobile devices such as smart phones or computer tablets comprising a wireless telecommunication technology (WTT) transmitter to connect remotely to the server system. The patient care system may advantageously further comprise a medical device connection station comprising a wireless telecommunication technology (WTT) transmitter configured for connection to the server system via a wireless telecommunications network, the medical device connection station configured to interconnect to the medical device and to upload medical device usage data to the patient information management program application via the WTT remote service data upload application on the server system. Alternatively, the medical device may incorporate a wireless telecommunication technology (WTT) transmitter configured for connection to the server system via a wireless telecommunications network, the medical device configured to directly upload medical device usage data to the patient information management program application via a WTT remote service data upload application on the server system.

In an embodiment, a client side software application may be installed on a mobile user interface device (UID) such as a phone or computer tablet, configured to upload medical device usage data to the patient information management program application via the WTT remote service data upload application on the server system.

The adherence to the treatment regimen is represented by a first graphical representation, and the outcome report relating to a physiological state of a patient is represented by a second graphical representation, the first and second graphical representations having a common time scale and displayed simultaneously on a graphical display of a user interface device such that an operator can compare the adherence to the treatment regimen and the physiological state of the patient.

The patient care system may advantageously further comprise a notification services software component configured to transmit email and/or SMS (Short Message Service) notifications to patients and optionally other users of the system. Notifications may include any one or more of alarms, visit reminders, treatment regime information, health information, and drug information.

The patient care system may advantageously further comprise patient therapy services software components including tests, such as vision and walking tests, accessible or downloadable from the server system by users and especially patients, the software component configured to capture results of tests and feed them into the reports software component. Patient therapy services software components may also include training exercises such as cognition training and mobility training. In an advantageous embodiment, patient therapy services may further be configured to receive data from sensors relating to physiological measurements of the patient's measured physiological data, for instance data captured automatically by sensors and transmitted to the server system via the patient's user interface device or medical device, or by the sensing or training device. Physiological data may for instance include any one or more of body temperature, blood pressure, pulse rate, weight, height, calories spent, galvanic skin response, surface electromyography, electroencephalography, oculography, breathing activity, muscle movement activity, blood sugar level, and other measurable parameters relevant to the medical condition being monitored.

Disclosed herein is a method of monitoring treatment and providing care to a patient suffering from a chronic disease or condition, comprising:
- providing a medical device for administering a treatment drug to the patient,
- providing a computerized patient care system comprising a server system configured to receive and transmit data via a communications network to and from users including patients and health care professionals, the server system comprising a database configured to store data related to the patient, an application server including patient care software components for neurodegenerative disease management and patient information management, a communication server for data transfer through the communications network,
- processing and storing data related to patient care on the server system,
- transferring medical device usage data comprising data on the usage of said medical device through the communications network to the server system, and
- processing said medical device usage data in conjunction with patient data to generate a report or a plurality of reports related to the treatment of the patient,
- providing remote access to the reports via the communications network to registered users of the patient care system as a function of respective roles and privileges of the registered users stored in the server system.

The method may advantageously comprise:
- providing a client side software application installable on a mobile user interface device (UID), such as a phone or computer tablet,
- uploading medical device usage data to the patient information management program application via the WTT remote service data upload application on the server system.

The method may advantageously comprise:
- uploading medical device usage data directly from the medical device to the patient information management program application via a WTT remote service data upload application on the server system.

The method may advantageously comprise:
- generating a report by the reports component software, in a form selected from any one or more of tables, charts, lists, diagrams or graphical representations, said report based on information selected from any one or more of drug administration history, adherence to treatment regimen data, patient outcome reports, patient health reports, patient physiological data reports, medical device settings, treatment regimen data, and any combination of aforesaid information.

The method may advantageously comprise:
- generating a composite report made of two or more reports for simultaneous display on a user interface device (UID) display, including composite adherence to treatment regimen and patient outcome reports to facilitate evaluation of the effects of non-adherence to the treatment regimen.

The adherence to the treatment regimen may advantageously be represented by a first graphical representation, and the patient outcome report relates to a physiological state of a patient and is represented by a second graphical representation, the first and second graphical representations having a common time scale such that an operator can compare the adherence to the treatment regimen and the physiological state of the patient.

Alternatively, the composite adherence report is displayed on a first time scale and the patient outcome report is displayed on a second time scale, wherein the first and second time scale are aligned and displayed simultaneously such that the composite adherence report and the patient outcome report are displayed on a common time scale, such that an operator can compare and/or correlate the adherence to the patient outcome report over a period of time.

Alternatively, the composite adherence report is displayed on a first time scale and the patient outcome report is displayed on a second time scale, wherein the first and second time scale are displaced in relation to each other, the patient outcome report being displayed on a common time scale with the composite adherence report and, such that an operator can compare and/or correlate the adherence to the patient outcome report over a period of time. An advantage is that for some health effects, there is a delay between the medication taken or missed and a measurable or perceived outcome effect of the patient.

The time scale may display adherence data and patient outcome reports per hour, day, week, month, year or any combination thereof.

The method may advantageously comprise:
- transmitting by means of the notification services software component, email and/or SMS (Short Message Service) notifications to patients and optionally other users of the system, notifications selected from a group including alarms, visit reminders, treatment regime information, health information, drug information.

The method may advantageously comprise:
- providing to patients online access to the tests,
- automatically capturing online results of said tests by the patient therapy services software components,
- feeding said test results into the reports software component and/or into the database.

The method may advantageously comprise:
- providing to patients online access to the training exercises selected from any one or more of cognition training, mobility training, speech training, vision training, cardiovascular exercise, physiotherapy.

The method may advantageously comprise:
- receiving data from sensors relating to physiological measurements of the patient's measured physiological data;
- transmitting said data from sensors to the server system via a communications equipped device selected from the patient's user interface device and/or medical device and/or by the sensing or training device, wherein physiological data is selected from any one or more of body temperature, blood pressure, pulse rate, galvanic skin response, surface electromyography, electroencephalography measurements, oculography measurements, electrocardiography measurements, breathing sensor measurements, blood sugar sensor measurements.

Also disclosed herein is a method for evaluating the efficacy of a treatment regimen for a chronic condition or disease comprising:
- providing a medical device to deliver a medicament,
- transmitting usage data of the medical device to a computing system
- calculating adherence to a treatment regimen based on a prescribed treatment regimen data and said usage data,
- transmitting to the computing system a patient reported outcome
- generating in the computing system a report comprising a first graphical representation of the adherence to a treatment regimen and a second graphical representation of the patient reported outcome, said first and second graphical representations comprising a common time scale
- rendering said report accessible to a health care professional for display on a screen of a user interface device, wherein said first and second graphical representations are simultaneously displayed.

An advantage of measuring the patient reported outcome is that a more complete picture of the patient's experience of life with diseases such as multiple sclerosis can be obtained, in view of the patient's perception of the effects of treatment and the disease course, thereby providing a quantifiable and broader measure of the impact of disease.

The computing system may advantageously comprise one or more of the features of a server system of a patient care system as described hereinabove.

In a particular aspect of the invention, the chronic condition or disease is a neurodegenerative disease including Multiple Sclerosis.

In a particular aspect of the invention, the chronic condition or disease is growth hormone deficiency.

Disclosed herein is a patient care system comprising: a medical device to deliver a medicament; a first data input device operable to acquire first data, said first data relating to the use of the medical device; a second data input device operable to acquire second data, said second data relating to a physiological state of a patient; at least one processing unit operable to communicate with the first and second data input device to acquire the first and second data, and operable to process the first data to create processed first data, wherein the processed first data relates to adherence to the treatment regimen; and a user interface device comprising a display unit operable to communicate with the processing unit and to display a first graphical representation representing adherence to the treatment regimen and a second graphical representation representing said second data, such that an operator can compare the adherence to the treatment regimen and the second data, wherein said second data comprises data collected from the patient in the form of responses to questions in a clinically validated questionnaire.

In an embodiment, the invention provides a computerised medical system comprising: a medical device for administering a medical treatment to a patient, a server system, a transmitter arranged to communicate with the medical device and to transmit first data to the server, said first data relating to the use of the medical device, a first computer terminal arranged to transmit second data to the server, said second data relating to the health of the patient, the server being arranged to store said first and second data, and a second computer terminal arranged to communicate with the server system and to simultaneously display a first graphical representation representing an adherence to the medical treatment deduced from said first data and a second graphical representation representing said second data.

Also disclosed herein in relation to an embodiment of the invention is a method to monitor the efficacy of a treatment regimen comprising: transmitting first data to a processing unit, said first data relating to the use of a medical device; transmitting second data to a processing unit, said second data relating to a physiological state of a patient; processing data to create processed first data, wherein the processed first data relates to adherence to the treatment regimen; and displaying a first graph representing adherence to the treatment regimen and a second graph representing said second data on a display unit such that an operator can compare the adherence to the treatment regimen and the second data.

The processing unit may be in a remotely accessible server system.

The first data may include data selected from: the type of medication administered, the dose of medication administered, the mode of administration, the time of administration, the date of administration and the frequency of administration. A processing unit may be provided to process the first data between its acquisition by the first data input device and its display by the display unit of a user interface device to represent adherence to a pre-determined treatment regimen. The second data may include data selected from: patient reported outcomes (PROs), health test results and physiological data.

Preferably, the system is operable to time stamp the first data by means of a clock module such that the time and/or date of use of the medical device is recorded. Preferably, the system is operable to time stamp the second data by means of a clock module such that the time and/or date of the physiological state of the patient is recorded. Ideally, both the first data and the second data are time stamped. In a preferred embodiment, the first data input device time stamps the first data and the second data input device time stamps the second data. The term "time stamped" refers to logging the time and/or date that the data was input. For example, the first data input device may be operable to log the time and/or date of each medical administration. Optionally, the second data input device may be operable to log the time and/or date of each patient reported outcome (PRO), health test result and physiological data.

Preferably the display unit is operable to display the first graphical representation and the second graphical representation on a common time axis. A time axis is a plot of time. Advantageously, this enables a user, in particular a health care professional, to determine whether or not there is a correlation between the processed first data and the second data over a defined time course. The correlation can be used to monitor the efficacy of a treatment regimen, such as a dosing regimen. For example, the user may be able to determine whether or not adherence to a treatment regimen has a positive effect on a physiological state of a patient. In certain embodiments, the treatment regimen is pre-determined. Preferably, the user interface device (UID) is operable to display on the display unit the first graphical representation and second graphical representation simultaneously.

Preferably, the user interface device display unit is operable to superimpose the first graphical representation and the second graphical representation. Advantageously, this enables the operator to view the first and second graphical representations together. As such, a possible correlation between the first and second graphical representations can be observed quickly and clearly.

Preferably, the UID display unit is operable to display the processed first data as a first graphical representation. Preferably, the display unit is operable to display the second data as second graphical representation. Advantageously, this enables the operator to observe changes in the processed first data and/or changes in the second data. Optionally, the processed first and second data is time stamped. In this embodiment, the processed first and second data can be plotted on a graph having a time axis. Ideally, the processed first and second data is plotted on a corresponding time axis.

The first graphical representation may be superimposed in front of or behind the second graphical representation.

The user interface device on which the graphical representations are displayed may be physically and geographically separated from other devices selected from: the processing unit, the medical device; the first data input device and the second data input device. The term "physical separation" means that no physical contact occurs between one or more items. The term "geographical separation" means that one or more items are located in different geographical places, such as different buildings, places, villages, towns, cities or counties. In general, the terms "separate" and "separated" refer to physical separation and optionally geographical separation. Physically and geographically separating the display unit from the above mentioned one or more devices is advantageous because it allows the data to be reviewed by an operator who is not the patient, wherein the operator is geographically separated from the patient. The operator may be a care giver, such as a healthcare practitioner or a field nurse or a healthcare payer, e.g. a health care insurance company. The healthcare practitioner may be a doctor or a nurse.

The UID with display unit may be a mobile phone (e.g. a smart phone) or a computer (e.g. a digital tablet or a PC) that is operated by the care giver. Typically a field nurse may compare the first and second data on a mobile phone. Typically the doctor may compare the processed first and second data on a PC in a doctor's surgery. Typically the health care insurance company may compare the processed first and second data on a PC in an insurance office. By comparing the processed first and second data, the care giver may determine whether or not there is a correlation between the processed first data and the second data. The correlation can be used to monitor the efficacy of a treatment regimen. The operator of the display unit may also be the patient. In this embodiment the display unit may be physically separated from devices selected from: the processing unit, the medical device; the first data input device and the second data input device. Alternatively, the display unit, the first data input device and the second data input device may be one and a same device. In a further variant of this embodiment, the display unit, the medical device, the first data input device and the second data input device may be one and a same device. Advantageously, the patient can compare the processed first and second data on a display unit, wherein the display unit is selected from a mobile phone (such as a smart phone) or a computer, such as a PC or a digital tablet. By comparing the processed first and second data, the patient may determine whether or not there is a correlation between the processed first data and the second data. The correlation can be used to monitor the efficacy of a treatment regimen.

The processing unit may be physically and geographically separated from other hardware components of the system, including the medical device, the first data input device, the second data input device and the user interface device comprising a display unit. Advantageously, this allows the first and second data to be stored and/or processed on the processing unit in a secure location. This improves the security of the data. In a preferred embodiment, the processing unit is in a server system.

Preferably the first data, processed first data and second data is encrypted. Advantageously, this improves the security of the data.

In an embodiment, the processing unit is operable to communicate with the first data input device and/or second data input device and/or the UID with display unit via one or more medical device connection stations comprising wireless telecommunication transfer technology (WTT). In an embodiment the medical device connection station comprises a subscriber identity module card (sim card) for connection to a mobile phone network, such as the GSM (Global System for Module Communication) or the UMTS (Universal Mobile Telecommunications System).

In an embodiment the medical device connection station can be physically separated from other devices selected from: the processing unit, the medical device, the first data input device, the second data input device and the UID with display unit. The medical device connection station may be a base onto which the medical device can dock.

The medical device connection station may communicate with the medical device via infrared, radiofrequency or electrical communication. As such, the first and/or second data can be communicated from the medical device to the medical device connection station via infrared, radiofrequency or electrical communication. In an advantageous embodiment, the medical device connection station is a WTT transmitter enabling transmission of data to the processing unit via a mobile phone communications network..

In an embodiment, the medical device comprises one of the at least one processing unit operable to generate the first and second graphical representations.

In an embodiment, the medical device connection station connects to a user interface device in the form of a computer, such as a patient's PC or digital tablet. The connection may be wireless or it may be a wired connection, such as a USB connection. The transmitter may transmit the first and/or second data to this computer. The computer may then transmit the first and/or second data to the processing unit. The computer may transmit the data via an internet connection.

In an embodiment, the first data input device is incorporated in the medical device, whereby the medical device can acquire the first data. The medical device can record data selected from: type of medication administered, dose of medication administered, mode of administration, time of administration, date of administration and frequency of administration, and optionally compute therefrom adherence to a pre-determined treatment regimen. The medical device may communicate with the processing unit in the server system via a medical device connection station. Alternatively, the medical device may incorporate a wireless transmitter, in particular a WTT transmitter.

The second data input device may be a mobile phone (e.g. a smart phone) or a computer, such as a PC or a digital tablet. A patient can input the second data to into their mobile phone or computer, wherein the second data relates to a physiological state of the patient. In this embodiment, the medical device is physically separate from the second data input device (e.g. the mobile phone or computer). Advantageously, by using a mobile phone or a computer as a second data input device, the medical device requires less hardware and is therefore lighter and smaller as compared to if the medical device and the second data input device were one and a same device.

In an alternative embodiment, the first data input device, the second data input device and, optionally, the transmitter are incorporated in a single device. Preferably, this single device is a mobile phone (e.g. a smart phone) or computer. The computer may be a PC or a digital tablet. In this embodiment, the medical device is physically separate from the first data input device, the second data input device and the transmitter (e.g. the mobile phone or computer). Advantageously, this allows for the medical device to be more easily disposable, for instance for single-use administration. Advantageously, the first and second data can be input into the same user interface device.

In an embodiment, the user interface device, such as a mobile phone or computer, is configured to communicate with the medical device, such as a disposable single-use medical device, via a label borne by the medical device. Ideally, the label is a Near Field Communication (NFC) chip and/or a Quick Response (QR) code. The medical device can be brought into contact or near contact with the mobile phone or computer equipped with an NFC transceiver, respectively QR code reader. This would trigger identification of the label by the phone or computer. Advantageously, the mobile phone or computer is operable to identify an individual medical device. Thus, the mobile phone or computer may acquire data selected from: the first data, the second data and data identifying the medical device and may transmit this data to the server system.

In an alternative embodiment the medical device, the first data input device and the second data input device are incorporated in a same device. As such, the medical device may be operable to acquire the first data and the second data. Advantageously, the patient need only input data into a single device. This would be beneficial to patients who do not wish to use a mobile phone or a computer. The medical device may also comprise a transmitter, whereby the medical device is operable to transmit the first data and second data to the processing unit physically or geographically separated from the medical device. Alternatively, the transmitter may be a base onto which the medical device can dock. The transmitter may advantageously be a WTT transmitter. Further optionally, additional second data, such as health test results, may be acquired by a further second data input device, such as a computer belonging to the patient.

The medical device may be a device for delivering medicament to a patient. The medical device may be an injection device, such as a subcutaneous injection device, an intravenous injection device or an intramuscular injection device. The medical device may be an electronic medical device or a mechanical device. Preferably, the medical device is an electronic subcutaneous injection device.

Alternatively, the medical device may be a pill dispenser, an inhaler or a topical administrator, such as a spray dispenser.

In a particular aspect of the invention, the patient care system, the method for monitoring the efficacy of a treatment regimen or monitoring treatment is configured for a disease selected from the group of multiple sclerosis, growth hormone deficiency, rheumatoid arthritis, juvenile rheumatoid arthritis, psoriasis, plaque psoriasis, Crohn's disease, juvenile Crohn's disease, asthma, psoriatic arthritis, ulcerative colitis, systemic lupus erythematosus and ankylosing spondylitis.

The term "data" is used to describe the first data and/or the second data and/or processed first data. Optionally, the term "data" may also be used to describe the third data and/or the fourth data, which are further defined below.

### The first data

In an embodiment, the first data relates to data selected from: type of medication administered, dose of medication administered, mode of administration, time of administration, date of administration and frequency of administration.

In a preferred embodiment of the invention, the system comprises a processing unit that is operable to process the first data. In a preferred embodiment of the invention, the method further comprises processing the first data to produce processed first data. The processed first data relates to adherence to a pre-determined treatment regimen.

The system or method of the invention may further comprise third data. Said third data comprises a pre-determined treatment regimen. The third data may comprise data selected from: pre-determined type of medication, pre-determined dose of medication, pre-determined type of administration, pre-determined time of administration, pre-determined date of administration and pre-determined frequency of administrations.

The processed first data may comprise data relating to adherence to a pre-determined treatment regimen, this may be referred to as treatment regimen adherence data. The treatment regimen adherence data is a correlation between pre-determined treatment regimen data and actual treatment regimen data. The pre-determined treatment regimen may be pre-determined by a care giver, such as a doctor. In an embodiment, the display unit displays the processed first data (treatment regimen adherence data) and the second data (a physiological state of the patient).

Thus, the processing unit is operable to calculate the adherence to a pre-determined treatment regimen by processing the first data. The processed first data is calculated by comparing the first data to the third data. In other words, the adherence to a pre-determined treatment regimen is calculated by comparing the actual treatment regimen data to the pre-determined treatment regimen data.

A pre-determined treatment regimen may be referred to as a prescribed treatment regimen.

Thus, an adherence of 100% indicates that the patient has completely adhered to the pre-determined treatment regimen, while an adherence lower than 100% indicates that the patient did not completely adhere to the pre-determined treatment regimen, for example the patient may not have injected all prescribed doses.

The UID display unit may display the processed first data as a percentage of adherence to the third data (i.e. the pre-determined treatment regimen). The percentages may be displayed as a graph. Thus, in one embodiment, the display unit displays a processed first data graph.

Preferably, the UID display unit is operable to display the processed first data and the second data graphically on a common time axis. Advantageously, this enables the operator to determine whether or not there is a correlation between the adherence to a pre-determined treatment regimen and a physiological state of a patient. Ideally, the UID display unit is operable to display the processed first data and the second data simultaneously, such as side by side or superimposed upon each other.

The pre-determined treatment regimen (i.e. the third data) may comprise data selected from: pre-determined type of medication, pre-determined dose of medication, pre-determined mode of administration, pre-determined time of administration, pre-determined date of administration and pre-determined frequency of administration. Thus, the pre-determined treatment regimen data contains time and/or date information.

The actual treatment regimen data (i.e. first data) may comprise data selected from: type of medication administered, dose of medication administered, type of administration, time of administration, date of administration of medication and frequency of administration of medication. The actual treatment regimen data (i.e. first data) may be time stamped, e.g. by a clock module. Processing of this time stamped first data results in time stamped processed first data.

The processing unit is operable to compare the time stamped first data to the time/date information contained within the third data.

The mode of administration may be injection such as subcutaneous injection, intravenous injection, intramuscular injection, preferably the mode of administration is subcutaneous injection. In an alternative embodiment, the mode of administration is oral or topical administration.

The first data input device may be a UID in the form of a mobile phone comprising a smart phone application or a computer comprising a web based application, wherein the application is operable to acquire the first data.

A dosing regimen is a form of treatment regimen.

### The second data

The second data comprises data selected from: patient reported outcomes (PROs), health test results and physiological data. The second data may advantageously comprise a PRO.

Patient reported outcomes (PROs) are responses to a clinically validated questionnaire. The questionnaire may contain questions permitting evaluating the level of criteria selected from general health, pain, cognition, fatigue, bladder condition, bowel condition, sexual satisfaction, visual impairment, mental health, feelings and emotions, depression, sleep and other health criteria of the patient. The questionnaire may require the patient to score one or more of the criteria on a sliding scale.

Health test include mobility tests, walking tests, vision tests and/or cognition tests. The tests may be done by the patient at home using a computer and test apparatuses such as motion sensors connected to the computer, e.g. a radiofrequency connection (e.g. Bluetooth® or Wi-Fi).

Physiological data includes, but is not limited to, body temperature, blood pressure, heart rate, galvanic skin response activity, breathing activity, blood sugar level, brain activity, eye movement activity, muscle movement activity, and height (in the case of a growth hormone treatment) of the patient. The system may comprise a height measuring device.

The second data is objective.

The second data input device may be a UID in the form of a mobile phone comprising a smart phone application or a computer comprising a web based application, wherein the application is operable to acquire the second data.

### The third data

The medical device, computer, mobile phone or server may comprise third data. Said third data comprises a pre-determined treatment regimen. The third data may comprise data selected from: pre-determined type of medication, pre-determined dose of medication, pre-determined type of administration, pre-determined time of administration, pre-determined date of administration and pre-determined frequency of administrations. Advantageously, the medical device, computer or mobile phone may remind or instruct the user to carry out the pre-determined treatment regimen.

### The fourth data

Fourth data may also be entered into the medical device or UID, for instance a computer or mobile phone. Said fourth data comprises a date and time of an appointment with a care giver. Advantageously, the medical device or UID may be operable to provide an alert to remind a patient of their appointment.

### Data input

The first data may be manually input into the first data input device. For example, the first data input device may have a user interface such as a touch screen, a keyboard or a computer mouse. The patient may input the data into the first data input device via the user interface. Preferably, the user interface is a touch screen. Ideally, the first data input device is a medical device or a mobile phone comprising a touch screen.

The first data may be detected by the first data input device. For example, the first data input device may be a medical device that is operable to automatically acquire data selected from: type of medication administered, dose of medication administered, mode of administration, time of administration, date of administration and frequency of administration.

The second data may be manually input into the second data input device. For example, the second data input device may have a user interface such as a touch screen, a keyboard or a computer mouse. The patient may input the data into the second data input device via the user interface. Preferably, the user interface is a touch screen. Ideally, the second data input device is a medical device or a mobile phone having a touch screen.

The second data may be detected by the second data input device, e.g. by a sensor. The sensor may be selected from: an electroencephalography (EEG) sensor, an electrocardiography (ECG or EKG) sensor, a skin surface sensor, an electro- or video-oculography sensor, breathing sensor, blood sugar sensor, a motion sensor, a vision sensor and a height sensor. The sensor may transmit data to the second data input device via a radiofrequency connection (e.g. Bluetooth® or Wifi). The second data may be input into multiple second data input devices.

### Data storage and processing

In a preferred embodiment, the system includes a data processing unit for processing the first data and/or the second data incorporated in a remotely accessible server system. The server system is operable to produce the first and second graphical representations and transmit them to the display unit.

In a preferred embodiment, the server system includes a database for storing the first data, processed first data and/or the second data.

In an embodiment, the server system is physically and geographically separated from the medical device and user interface devices (UID) of the system operating as the first data input device, the second data input device and the display unit. Advantageously, this allows the data to be stored and/or processed on the server in a secure location, the extent and type of information accessible by different users depending on their roles and functions. This improves global accessibility as well as the security of the data, and furthermore allows collection of data over time that may be used to gain a better understanding of the effects of treatment on the disease.

In an embodiment, the medical device comprises a data storing module operable to store the first and/or second data offline, which may then be transmitted to the server system at a time that is convenient to the operator of the medical device. Thus, data that has been collected over a period of time, such as days weeks or months, e.g. up to twelve months, can be transmitted simultaneously to the server system.

### Display of data

The UID display unit is operable to display the processed first data and second data to enable an operator to compare the processed first data and second data. Advantageously, this enables an operator to more conveniently determine whether there is a correlation between the processed first data and second data.

In a preferred embodiment, the processed first data can be displayed as a diagram, preferably a first graph. Likewise, the second data can be displayed as a diagram, preferably a second graph. Advantageously the first and second graphs can be displayed simultaneously. For example the first and second graphs can be displayed next to each other or superimposed over one another. Alternatively, the processed first data and second data can be displayed in the form of a calendar, such as a daily, weekly or monthly calendar.

### Telecommunication Networks

Data is transmitted within the system via telecommunication networks selected from: a wireless telecommunication technology (WTT) network, also known as a mobile or cellular phone network, a fixed line telephone network, an internet network and a local computer network such as an intranet network including various communication protocols and means such as WiFi and Ethernet.

In many embodiments, data can be transmitted over one or more of these networks. For example, the first data input device may transmit first data to a computer via a mobile phone network. The computer may then transfer the data to the server system by an internet network. For example, the second data input device may transmit second data to a server via a mobile phone network. For example, the server system may transmit processed first data and second data to a display unit via an internet network.

Advantageously, the transmission of data between devices may be wireless.

### Medical devices

The medical device is for administering medical treatment to a patient, in particular for administering a medical treatment drug. In one aspect of the invention, the medical treatment is treatment for multiple sclerosis, such as interferon beta-1a, e.g. Rebif® or Avonex®. In another aspect of the invention, the medical treatment is treatment for growth hormone deficiency, such as recombinant growth hormone, e.g. Saizen®.

In an embodiment, the medical device is an injection device, such as a subcutaneous injection device, an intravenous injection device or an intramuscular injection device.

Preferably, the device is an electronic subcutaneous injection device, for instance of a type commercially known as Rebismart®, Rebidose®, or Easypod®. Rebismart® administers Rebif® for treatment of multiple sclerosis. Rebidose® is a disposable medical device which administers Rebif® for treatment of multiple sclerosis. Easypod® administers Saizen® for treatment of growth hormone deficiency.

In an embodiment, the medical device for multiple sclerosis treatment incorporates the first data input device. Advantageously, this means that the medical device for multiple sclerosis treatment can acquire the first data. In this embodiment, the medical device for multiple sclerosis treatment can record data selected from: adherence to a pre-determined treatment regimen; type of medication administered, dose of medication administered, mode of administration, time of administration, date of administration and frequency of administration.

The medical device for multiple sclerosis treatment may communicate with a server via a transmitter.

Preferably, the transmitter is a base to which the medical device for multiple sclerosis treatment is docked. The medical device for multiple sclerosis treatment may be operable to communicate with the transmitter by infrared, radiofrequency or electrical connection. Ideally, the transmitter is a wireless transmitter.

The medical device for growth hormone deficiency treatment may operate in the same way as the medical device for multiple sclerosis treatment.

The medical device for multiple sclerosis or growth hormone deficiency treatment may be used in conjunction with a second data input device. The second data input device may be a mobile phone or a computer, such as a PC or a digital tablet comprising a client side software application for capture and processing of the data. In this embodiment, a second transmitter and the mobile phone or computer are incorporated in a same device. As such, a patient can input the second data to into their mobile phone or computer, wherein the second data relates to a physiological state of the patient. In this embodiment, the medical device for multiple sclerosis or growth hormone deficiency treatment is physically separate from the second data input device (e.g. the mobile phone or computer). Advantageously, by using a mobile phone or a computer as a second data input device, it means that the medical device for multiple sclerosis or growth hormone deficiency treatment requires less hardware and are therefore lighter and smaller than as compared to if the medical device and the second data input device were one and a same device.

In an alternative embodiment, the medical device for multiple sclerosis or growth hormone deficiency treatment is disposable and physically separate from the first data input device, the second data input device and the transmitter. In this embodiment, the first data input device, the second data input device and the transmitter may be incorporated in a same device, such as a mobile phone or a computer comprising a client side software application for capture and processing of the data. The first and second data can be input into the same mobile phone or computer which improves convenience for the user.

Preferably, the mobile phone or computer is arranged to communicate with the disposable medical device for multiple sclerosis or growth hormone deficiency treatment via a label borne by the disposable medical device. Ideally, the label is a Near Field Communication chip and/or a Quick Response code. The disposable medical device can be brought into contact or near contact with the UID in the form of a mobile phone or computer. This would trigger identification of the label by the phone or computer. Advantageously, the mobile phone or computer comprises a client side software application operable to identify an individual the disposable medical device. Thus, the mobile phone or computer may acquire: the first data, the second data and data identifying the medical device, and then transmit this data to the server system.

Other features and advantages of the present invention will become apparent from the reading of the following detailed description made with reference to the appended drawings.

### Brief description of the drawings

Figure 1 is a schematic overview illustration of a patient care system according to an embodiment of the invention;
Figure 2 is a schematic overview illustration of a server system of a patient care system according to an embodiment of the invention;
Figure 3 is a schematic overview illustration of a functional architecture of a server system of a patient care system according to an embodiment of the invention;
Figure 4 is a schematic overview illustration of a software system context diagram of a server system of a patient care system according to an embodiment of the invention;
Figure 5 is a schematic overview chart of software components of a patient care system according to an embodiment of the invention;
Figure 6 shows a screen interface illustrating a composite report that a physician may use to analyse patient adherence to treatment, according to an embodiment of the invention
Figures 7a to 7b are illustrations of medical devices according to embodiments of the invention, figure 7a illustrating a reusable medical device docked on a medical device connection station and figure 7b illustrates a single-use disposable medical device;
Figures 8a to 8c are simplified schematic block diagrams illustrating an aspect of an embodiment of a patient care system according to the invention.

### Detailed description of exemplary embodiments

In the following description, in exemplary embodiments of the present invention the patient care system and method for monitoring or evaluating the efficacy of treatment are configured for treatment of multiple sclerosis or growth hormone deficiency. The present invention may also be applied in treatments for other diseases such as rheumatoid arthritis, juvenile rheumatoid arthritis, psoriasis, plaque psoriasis, Crohn's disease, juvenile Crohn's disease, asthma, psoriatic arthritis, ulcerative colitis, systemic lupus erythematosus and ankylosing spondylitis.

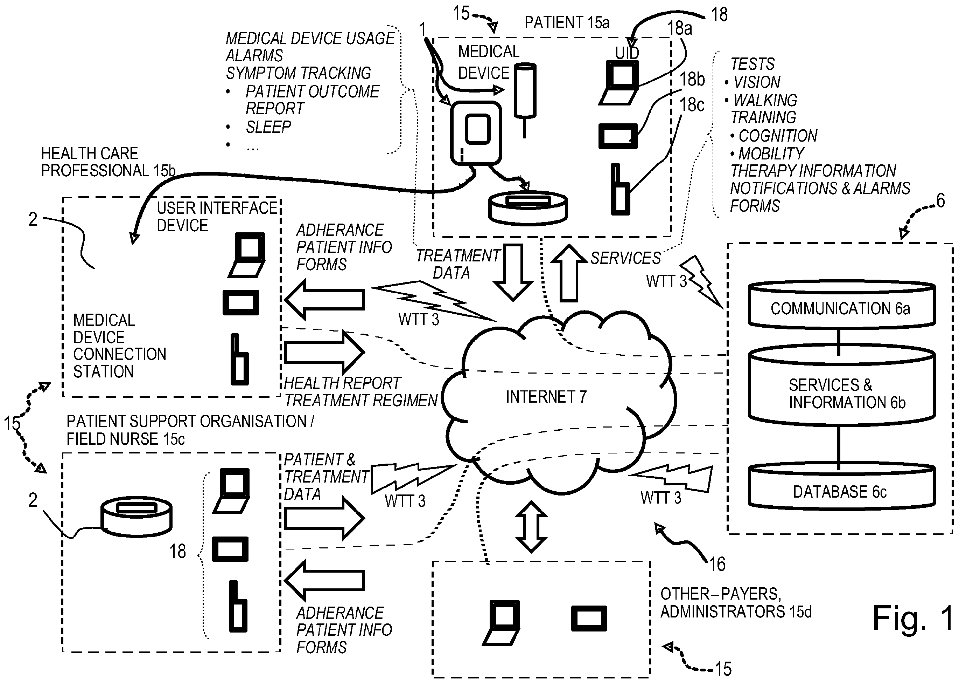

Referring to Figure 1, a patient care system according to the invention comprises a medical device 1 for administering a medical treatment to a patient, a server system 6, a user interface device 18, and depending on the embodiment, a medical device connection station 2. The server system 6 is configured to process and/or store information related to patient care and accessory services, such information being received and transmitted via a communications network 16, in particular a global computer network such as the internet 7. The communications network may further include a wireless telecommunications transfer (WTT) network, for instance a mobile phone network 3, and direct point-to-point communication between users 15 and the server system 6.

Users include patients and health care professionals (HCP), in particular physicians and nurses. Users may further include patient support organisations or persons (PSO), for instance including field nurses. Users may further include one or more of system administrators, health insurance organisations, suppliers of health services, manufacturers and suppliers of medication, pharmacies, and payers of health services. HCPs manage the patient related information in the system. This role creates patient entries, and manages and monitors the patients' related data in the system to enable better treatment and care to the patients. A system administrator is responsible for managing the system related functions in the applications that includes creating users, roles and functionalities, and other master data. The role of a field nurse may be similar to that of the HCP and can manage all patient data. The field nurse may typically access the application using a mobile user interface device, such as a computer tablet or smartphone. A field nurse may for instance schedule appointments, meet the patients, and collect information from the patient.

User interface devices may comprise a display and means to communicate with the server system 6 or the medical device connection station, and may include personal computers 18a, computer tablets 18b, mobile phone devices 18c or other electronic computing devices with a graphical user interface and means to communicate with the server system 6 or the medical device connection station.

A user interface device may be integrated in the medical device 1 such that, depending on the embodiment, the patient does not require a separate user interface device to connect to the server system or the medical device connection station. Client application programs may be installed on the medical device and/or the user interface device, the client application program configured to establish connection with the server system 6 or medical device connection station 2 and to manage the transmittal and reception of data to, respectively from, the server system or medical device connection station.

The medical device connection station 2 may have different configurations depending on the variant. In a variant, the medical device connection station 2 may be configured as a communication interface device that serves to communicate information to and from the medical device to a user interface device, for instance the user interface device of a health care professional 15b, and/or through the communications network 16 to and from the server system 6. The medical device connection station may, in another variant, further comprise a processor and software configured to process raw data from the medical device in an information relevant form to a user 15 via the user interface device, or to the server system 6. The medical device connection station comprises a connection interface complementary to a connection interface of the medical device. The connection interface may include direct electrical contact, for instance a USB connection, or various wireless communication systems, in particular near field communication systems, which may operate with various known communication protocols such as infrared, Bluetooth, Zigbee and other radio frequency (RF) communication protocols.

Within the scope of this invention, medical devices include reusable injection devices, disposable injection devices, implantable drug delivery devices, intradermal disposable devices, and electronically readable packaging of solid form drugs, for instance pills for oral delivery, or suppositories, in electronic container or blister packaging which senses when a drug dose is removed from the container or packaging.

Data transmission between the server system 6 and users 15, via their medical device 1, and/or medical device connection station 2, and/or user interface device 18 is preferably encrypted, for instance using a public key / private key pair encryption means, a symmetric encryption means, or other encryption means known per se.

The data are stored confidentially in the server system 6 and processed and classified so as to be easily and securely accessible to the various users 15a, 15b, 15c, 15d as a function of the access rights allocated to each type of user and the identity of the user. Medical information on the patient is accessible to the patient 15a and the physician 15b, possibly also to authorized members of the patient support organisation 15c, such as field nurses, but not to other users.

The server system 6 may comprise a plurality of physical and/or virtualised servers in a single location or in a plurality of locations distributed in the communications network 16. The meaning of "server" thus encompasses hardware servers with server software dependant on the hardware server configuration, and virtualised servers installed on one or more hardware server components that are independent of the hardware server configuration. A server may also be formed by a plurality of server hardware and/or software components that are distributed in a computer network.

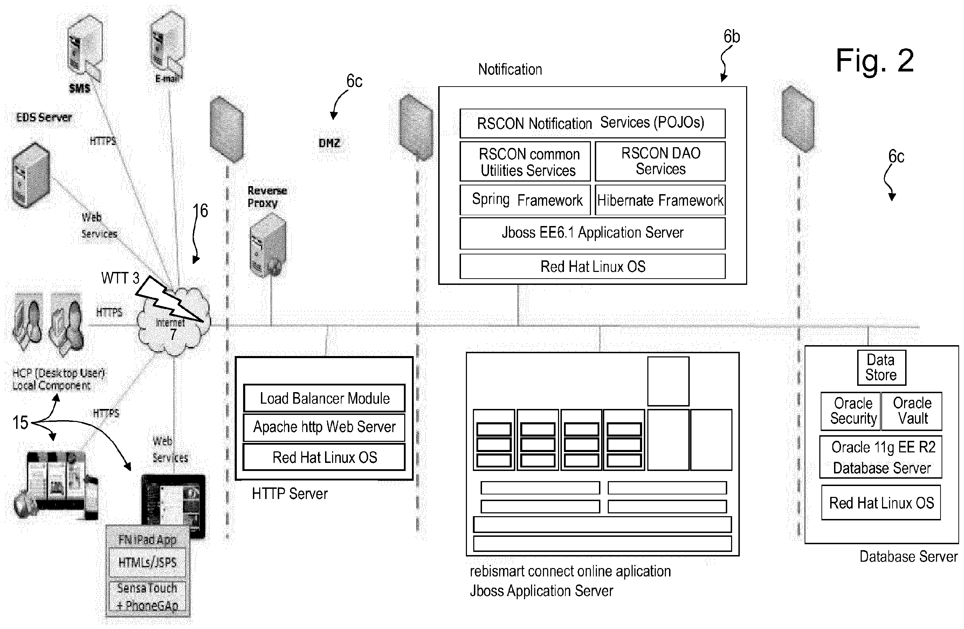

Referring to figures 1 and 2, in an advantageous embodiment, the server system comprises a communications server system 6a including a web server (HTTP server), an application server system 6b, and a database 6c. The application server system may comprise an information server for handling online services accessible by the users, a notification server for handling notification services to users, a server for handling mobile components, and a server for handling data load services from local components and devices.

### Example of a server system

In an example, a server system according to this embodiment may comprise the following features:

### HTTP (Hvper Text Transfer Protocol) Servers:

- May be Apache HTTP Web servers in a demilitarized zone protected by a firewall that will only allow HTTPS or HTTPS SOAP requests. No other protocol is allowed to hit the HTTP servers. All requests from user devices are through SSL (secure socket layer) to ensure that all communication between the user devices and HTTP servers is encrypted during transmission.
- No patient or medical data will reside on the HTTP Servers
- The HTTP servers are load balanced to provide performance and high availability. If one of the Web servers goes down for any reason, the performance of the system in case of peak workloads might be impacted but the system will still be available, and able to handle user requests.

### Application Servers:

- May be a JBoss Application Server clustered to provide high availability
- JBoss clustering will handle session management

### Application Servers for Notification component:

- The JBoss application server hosts the notification component, for instance for handling notifications sent per email or SMS (short message service). This server does not need to be clustered since it will not receive requests from users.

### Application Servers for Mobile Components:

- The JBoss Application Server is preferably clustered to provide high availability

### Application Servers for Data Load Services from Local Component and Devices:

- The JBoss Application Server may be clustered to provide high availability

### Database:

- May for instance be an Oracle™ Enterprise Edition database that is clustered to provide high availability
- An Oracle Advance Security Option can be used to provide data encryption for stored data.

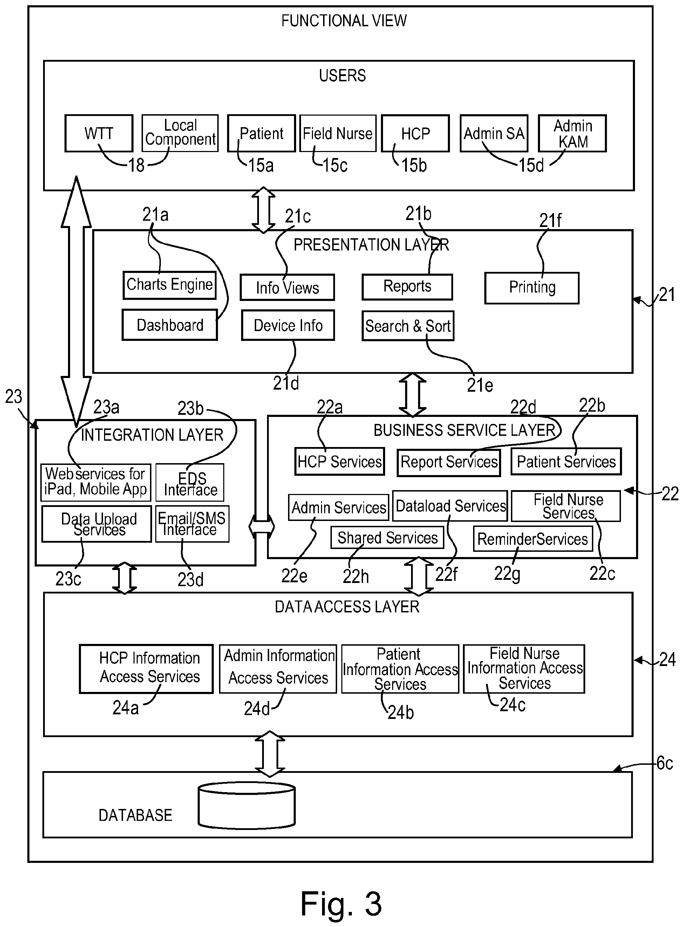

Referring to figure 3, a functional diagram of the program architecture of an exemplary embodiment of a computerized patient care system according to the invention is illustrated. The program functions comprise presentation program components 21, business services program components 22, integration program components 23, and data access program components 24.

The presentation components are responsible for managing the requests and response from the user's interface device, for instance from a network browser on the user's interface device, and rendering the presentation to the respective users. The presentation components are specialized to render charts 21a, reports 21b, views 21c, device information 21d, data entry and search 21e, and prints 21f.

The business services components are responsible for providing all the functionality required for the HCP and administrator users. They hold the business logic to process and provide the data back and forth to the users. The business services components are grouped based on the functionality they provide to the users, and include HCP services 22a, patient services 22b, field nurse services 22c, report generation services 22d, administrative services 22e, data load services 22f, reminder services 22g, and shared services 22h. The shared services provide functionality, such as logging, error handling, audit trail, caching, security, and notification used by all the other program modules.

Integration Components are responsible for providing the interfaces to various external interface devices, including web services for mobile applications 23a, EDS interface 23b, data upload services 23c, email and SMS interface 23d. These program components may include:
- programs to upload device data from the local component and the WTT system
- Web-services to provide patient and associated data to mobile applications (e.g. iPad™ application)
- Web-services to provide field nurse and associated data to mobile applications (e.g. iPad™ application)
- Integration with EDS (Enterprise Data Services)
- E-mail gateway integration
- SMS gateway integration

The data access components provide functionality to manage data storage and retrieval from the database, and include HCP information access services 24a, patient information access services 24b, field nurse information access services 24c and administrator information access services 24d.

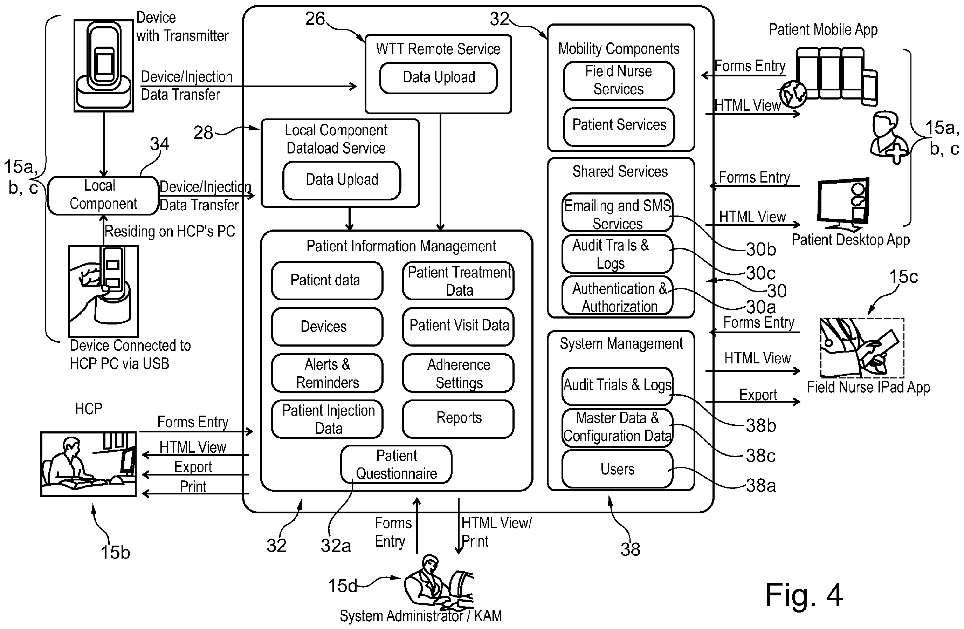

Figure 4 illustrates an exemplary embodiment of the interactions between users and the server system on a contextual level. In this example, the various users, including patients 15a, health care professionals 15b, field nurses 15c, and administrators and key account managers (KAM) 15d access the software applications on the server system 6 remotely through the communications network 16, including in particular through the internet 7. Administrators and KAM and health care professional may typically access the system via a user interface device (UID) 18 in the form of a desktop computer for instance, whereas patients and field nurses may typically access the system through a desktop UID as well as a mobile UID in the form of a smart phone or computer tablet.

For the mobile UID's, medical device data may be uploaded into the server system 6 using a WTT remote service data upload application 26 in the server system, directly from the medical device through the wireless telecommunications network 3 using the available mobile cellular systems, for instance GPRS (general packet radio service), UMTS (Universal Mobile Telecommunications System) or others. UIDs accessing the server system through the internet 7 can also upload medical device data, in particular usage data stored in the medical device, through a local component dataload service application 28 on the server system.

Only registered users can access the server system. Users are authenticated by an authentication and authorization program application 30a forming part of a shared services section 30 available to all users. User data, including identity, type, and respective roles and privileges is stored in the server system, for instance in the database 6c, and enables users to have access to authorized functionality and data based on their respective roles and privileges, for instance patient sensitive data may be available only to HCPs and patients, whereas server system administration functions may be available only to system administrators, and forms configurations available only to key account managers and system administrators.

In the example illustrated, data may be inputted into the server system 6 in the following ways:
- The medical device 1, incorporating or connected to a WTT (Wireless Telecommunication Technology) transmitter in the medical device connection station 2 for direct upload of data, in particular medical device usage data, for instance injection history data, to the patient information management program application 32 via the WTT remote service data upload application 26 on the server system. The WTT module receives the data, and after successful validation stores it in the online database 6c.
- The medical device 1 device can be connected to a UID (user interface device) 18 either directly or via the medical device connection station 22, the UID comprising a local component program (a client side program) 34 for the transfer of information to the server system 6. The local component program 34 receives medical device usage data, for instance injection history data, and uploads it to the server system 6 via the local component data upload service application 28 over HTTPS (Hyper Text Transfer Protocol Secure) requests. The local component data upload service application 28 validates the uploaded data and stores it in the online database 6c. As an example, the local component can communicate with the program on the server system using a Spring™ framework http invoker feature, and the medical device communication station 2 may also communicate with the WTT remote data upload service application 26 via the Spring™ HTTP (Hyper Text Transfer Protocol) invoker.
- User input: HCPs, administrators, KAM (country based Key Account Managers), patients, and field nurses can log on to the server system and input various types of data fields into the server system through various forms available in the application. For instance patient information, medical device association information, and clinic information are entered by the users in the application. Sensitive patent information is stored in the database 6c in an encrypted form and is only accessible to authorized users, which may include HCPs and field nurses.

In an exemplary embodiment, in an online mode, the communication between the UIDs 18 and the various web accessible services on the server system 6 may be secured using the HTTPS protocol. In an offline mode, the medical device comprises a database (for instance SQLite) used to store the usage data locally such that user can save the medical device usage data and other data in the database of the device when the user is offline. This can use for instance a JavaScript encryption mechanism (e.g. AES 128) for the data in the local storage. The pages are available in the UID 18 in the offline mode. The user may require a password and User ID to log on to the application in the offline mode. This password maybe used as a key for the encryption/decryption of the data.

In the exemplary embodiment illustrated, the patient information management application 32 generates information in various formats for output of the server system 6, for instance including HTML views and forms for entering data, and various list and form views displaying information to the user about patient devices.

HTML view and forms may be further categorized as follows:
Various list and form views display information related to the patient for use by the patient, HCPs and field nurses and may include :
- *Profile* - *Edit*
- *Reminders*
   - Configuring Adherence Threshold Values
   - Schedule Reminders
- *Patient Reports*
   - History Overview Chart - allows the HCP to view graphical representation of injection history data for a selected patient.
   - Injection History List - HCP can view the injection data as list for a selected patient.
   - Injection History Graph - HCP can view the graphical representation of the injection data for a selected patient.
   - Injection history Calendar view - HCP can view the calendar representation of the Injection data for a selected patient.
   - Adherence Data Reports
   - Outcome Reports
   - Combinations of the above to form composite reports, most advantageously including composite adherence and outcome reports to evaluate the effects of non-adherence to the treatment regimen
- *Patient's Device*
   - Device Settings - view of current and history of device settings
   - Device Registration
   - Device Assigned/Un-assigned
- *Calendars* - *For Patient and field nurses only*
   - View day wise reminders, appointment, events
- *Visit and Remarks*
   - Add Visit and Remarks
   - Edit Visit and Remarks

Various list and form views displaying information to the user about patient devices may include:
- Device Management - This functionality allows user to assign and un-assign devices to the patient.
- View Active Device Settings - This view allows the user to view the settings of the device assigned to a specific patient.

Server system outputs may further include a printing function allowing users to print the viewable data and a restricted data export function, for instance:
- HCPs are allowed to export the patient's injection history data and device settings from the system, for instance as a comma-separated values file (CSV).
- Field nurses are allowed to download patient and appointment information on their UID 18, for instance their computer tablet 18b, for offline use during patient visits. This data is encrypted and stored in a local offline database on the UID (18).

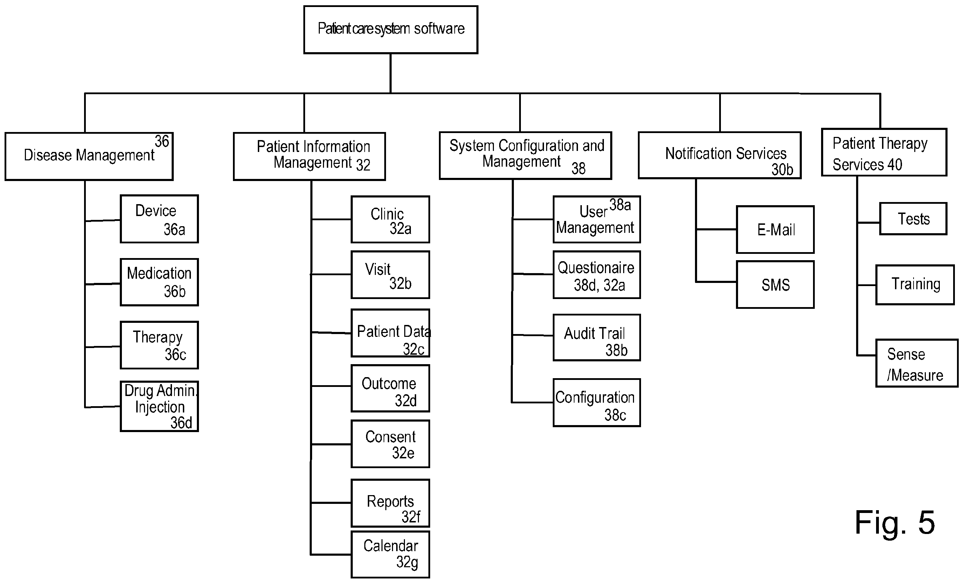

Referring to figure 5, in conjunction with figure 4, software components of the patient care system according to the invention may be categorized as follows:

### i. Disease Management 36

This category comprises software components which are used to manage the disease. In a particular embodiment of the invention, the disease is a neurodegenerative disease, in particular Multiple Sclerosis. These are software components that relate to the medical device configuration and use 36a, medication 36b, therapy 36c and drug administration 36d , e.g. injection.

### ii. Patient Information Management 32

This category comprises software components which are used to manage information on patients, in a particular embodiment on Multiple Sclerosis patients. These include information related to the treating clinic 32a, medical and therapeutic visits 32b, patient consents 32e, patient data 32c, treatment outcomes 32d, patient and HCP reports 32f, and calendar 32g software components.

### iii. System Configuration and Management 38

This category comprises software components which are used for configuration and management of features used across the patient care system. These include user management 38a, 30a, questionnaires 38d, including patient questionnaires 32a, and audit trail software components 38b, including shared services audit trail components 30c.

### iv. Notification Services 30b

This category comprises software components which are used for notification of events to users 15. These include e-mail and SMS software components 30b.

### v. Patient Therapy Services 40

This category comprises software components which are used to provide information to the patient inter alia to assist in improving therapy, disease management and patient reports. Patient therapy services software components include online tests 40a, such as vision and walking tests, online training 40b such as cognition and mobility training, disease information for patients and patient support organisations, and online patient physiological monitoring. Online patient physiological monitoring for multiple sclerosis may comprise a digitalised version of the 9-Hole Peg Test, which is a quantitative test of upper extremity function of a patient.

Depending on the type of disease to be treated, additional patient therapy services may include rehabilitation, stress monitoring, cognitive behavioural therapy, depression assessment and/or treatment, fatigue monitoring and treatment, activity tracking and gait assessment. Stress monitoring may for instance include measurements of heart rate and oxygen in the blood. Depression may be assessed and/or treated by the present system and device as a secondary disease in conjunction with e.g. multiple sclerosis, or as a primary disease.

Activity tracking may include information such as a physical exercise performed (distance walked, time and/or load), calorie consumption, and heartbeat. Gait assessment can be used as a computerised method for assessing, planning, and treating individuals with conditions affecting their ability to walk. Especially for patients having a disease which affects their ability to walk (e.g. multiple sclerosis), gait assessment can be used in order to assess, plan, and treat the disease. A gait analysis may include several cameras configured to record the position of markers attached to the patient's body. A computer calculates the trajectory of each marker in three dimensions and a model of the movement of the underlying bones can be calculated such that a complete breakdown of the movement of each joint can be determined.

Patient therapy services may further include alarms 40c, for instance alarms for administration of medication and alarms for visits to the treating clinic, the HCP, or the field nurse. The online capture of results of tests may be fed into the reports 32f component and the performance of training may also be recorded and available to the reports component or as information available to the HCP and filed nurse. Alarms may be sent by the notification services component 30b. Tests may be done by the patient at home using the patient's UID 18 and test apparatuses such as motion sensors connected to the UID 18 by a wired or wireless connection. The tests may be vision tests, walk tests, cognition tests and/or mobility tests. Patient physiological monitoring may also include physiological measurements of the patient's measured physiological data, such as body temperature, blood pressure, pulse rate, height (in the case of a growth hormone treatment) of the patient. Certain physiological measurements may be captured automatically by sensors and transmitted to the server system via the patient's UID 18 and/or medical device 1 an/or by the sensing or training device if it is equipped with communications means for data transfer over the internet 7 or the WTT network 3. Certain physiological data may be manually entered by the patient 15a or the HCP 15b or the field nurse 15c.

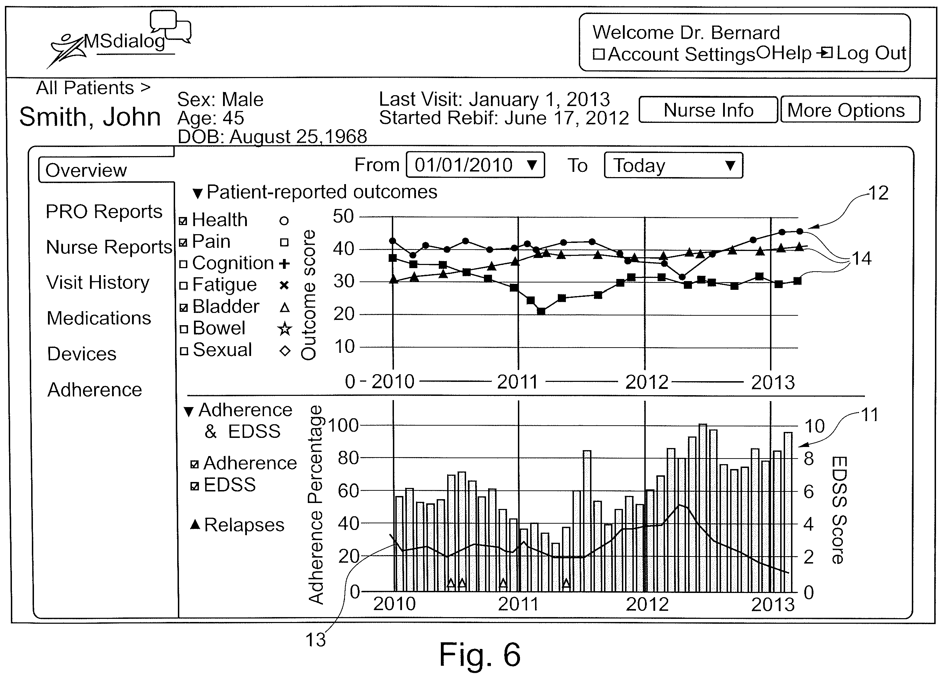

In an exemplary embodiment, the software components illustrated in figures 4 and 5 and presented above may comprise the following features:
*Therapy 36c* - this software component provides functionality related to drug therapy which uses medication to treat the disease, in a particular embodiment multiple sclerosis disease. Therapy is associated with medication as medication is governed by a Therapy.
*Medication 36b* - this software component provide functionality related to medication prescribed or taken by the Patient. Medication component is associated with Therapy as Therapy governs the Medication given to patients.
*Device 36a* - this software component provides functionality related to the medical device 1, in a specific embodiment the medical device is Rebismart™ used to inject Rebif™ medicine to treat multiple sclerosis disease. Device component 36a stores medical device usage data, for instance injection data, and is also associated with patient data 32b as every patient will have at least one device associated with him / her.
*Injection 36d* - this software component provides functionality to store the date and time of drug administration, for instance injections, and their related information (e.g. injected dose). In a specific embodiment, Injection is a method to give medicine to multiple Sclerosis patients. Injection data will be passed to Device component 36a.
*Patient Data 32c* - this software component provides maintenance of patient data which is gathered using Device component 36a and a web application.
*Clinic 32a* - this software component manages the association of a treating clinic to a patient 15a, in a specific embodiment a Multiple Sclerosis patient.
*Consent 32e* - this software component manages the consent of the patient 15a for being treated by a primary physician or other HCPs 15b whoever might be involved in treating the patient in the same clinic where patient is undergoing the treatment.
*Patient Outcome 32d* - this software component will provide the outcome of questionnaire presented to patient and will use Report component 32f for generating the health reports for a patient 15a, in a specific embodiment for a patient suffering from Multiple Sclerosis disease.
*Patient Visit 32b* - This software component manages the visit information of the patient entered by the HCP 15c and field nurse 15d. Visits help to track the EDSS Score, relapses and the hospitalization period of the patient since last visit.
*Reports 32f -* this software component generates patient reports from the data received through medical device usage received from the medical device that provides adherence data, questionnaires filled by the patient that may provide patient reported outcomes, adherence settings including information on the treatment regimen, and visit data included by the HCP and field nurse. Reports advantageously include composite diagrams that present in a single view multiple reports along a common time scale, including patient reported outcomes (PRO) and adherence information along a common time scale, as will be discussed in more detail further on and illustrated in figure 6. The afore mentioned composite diagram enables the HCP to more easily and quickly assess the effects of non-adherence of a patient to the prescribed treatment regime and if needed to propose corrective measures such as reminding the patient on importance of adherence, enhanced monitoring of adherence, increased frequency of visits, a modification in the treatment regime, and additional treatment or therapeutic measures.
*Calendar 32g* - this software component will manage calendars for HCPs and patients for providing details on events as well as scheduled visits. Calendar software component 32g may also issue reminders of an event to patient or HCP.
*Questionnaire 38d* - this software component manages all the questionnaires presented to the patient and their responses. This feature may provide outcomes of surveys provided by the patient information management module 32 to the patients. The questionnaires include clinically validated questionnaires directed to the patient, the responses of whom are used by the Reports component 32f to generate patient reported outcome (PRO) reports. The questionnaire may contain questions permitting evaluating the level of general health, pain, cognition, fatigue, bladder condition, bowel condition, sexual satisfaction or other health criteria of the patient.
*User Management 38a* - this software component involves managing user access rights of the various users (HCP, field nurse, patient, system administrator, key account manager and payer).
*Audit Trails 38b* - this software component provides audit trail management for all transactions executed in the patient care system by any user.
*Configuration 38c* - this software component is used for managing system configuration and TimeZone configuration e.g. country specific configuration.
*E-Mail and SMS 30b* - these software components send e-mail notifications, respectively SMS notifications to users of the patient care system.

Certain of the software components described herein may be in the form of client-server applications whereby a client side application is installed on the user interface device (UID) to allow the UID to connect to the application on the server system and run certain features locally. Certain software components described herein may be in the form of browser applications that are accessed and run through a web browser on the users UID. Certain software components may also be installed and run independently on various devices of the system including medical devices, medical device connection stations, and user interface devices, depending on the type and function of the software application. The use of the term "software component" herein may mean a portion of a program, a program, a plurality of portions of programs or a plurality of programs that work together to achieve the desired function.

The integration of the aforementioned software components in the server system 6 communicating with client applications installed on both mobile and wired user interface devices 18 described herein provides a flexible global patient care system optimising the quality of information available to patients, HCPs, field nurses and other members of patient support organisations as well as other actors such as drug manufacturers to improve overall patient care and health, while reducing the healthcare costs. Moreover, over time, data accumulated in the database allows the HCPs and drug providers to gain a better understanding of the disease and the effects of treatment in order to improve the treatment regime and therapy. The patient care system according to the invention allows remote patient treatment monitoring for health professionals, provides information for decision making to HCP's, enables ease of administering medications for patients, provides easy health communication between HCPs and patients globally, and provides a safe, effective and economical patient care.

In an embodiment of the invention, the medical device 1 is an injection device for injecting liquid medicine, in particular Rebif® (interferon beta-1a) or Saizen® (recombinant growth hormone). The medical device 1 may for example be of the type, or include certain features of, the devices disclosed in WO 2005/077441, WO 2006/085175, WO 2006/085204 or WO 2007/088444 (which are incorporated herein by reference) and marketed under the registered trademarks RebiSmart and Easypod.

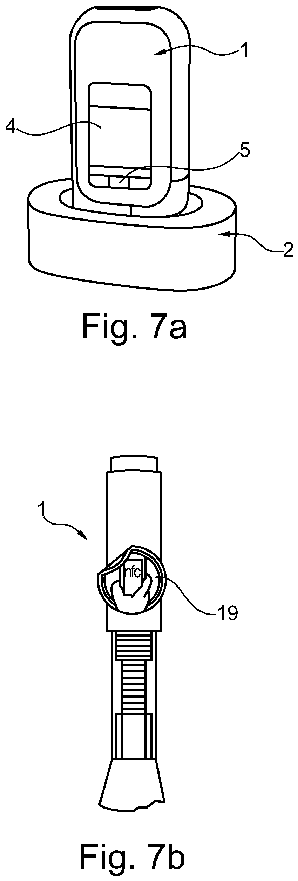

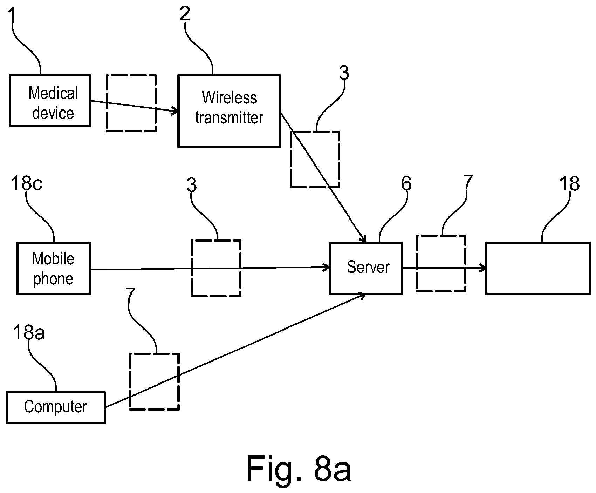

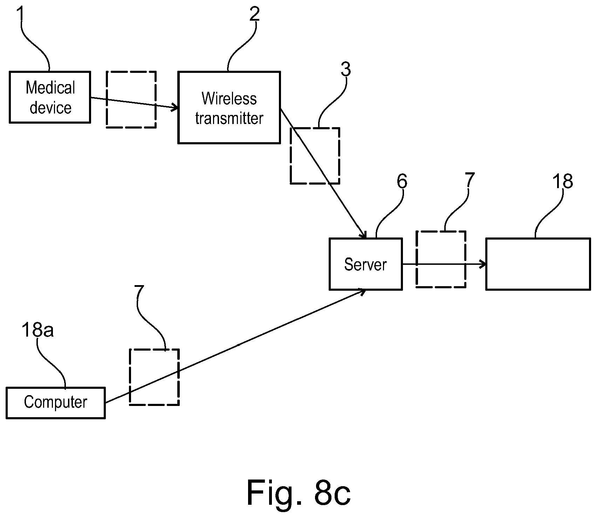

Referring more particularly to figures 7a, 7b, 8a to 8c an example of the configuration of a patient care system according to an embodiment of the invention, especially for monitoring adherence to a treatment regime, for instance a treatment regime for patients suffering from Multiple Sclerosis or growth hormone deficiency, will now be described.

In the embodiment, the medical device 1 preferably includes an internal memory and processor which is programmed to store first data relating to the use of the medical device by the patient in the memory.

The first data includes medical device usage data, comprising the amount of medication administered and the date and/or time of administration. In a variant where the medical device is an injection device, first data may include the number of injections made, the times of the injections, and the doses injected. The internal memory and processor further stores an identification code that uniquely identifies the medical device 1.

In variants where the medical device does not comprise and internal memory and microprocessor to store the medical device usage data, the patient or person administering the medication may manually enter the first data in a user interface device 18 provided with a client software program generating a form for entry of the data and storage of the first data in a memory of the user interface device.

In an embodiment, the system also comprises a medical device connection station 2. As shown in Figure 7a, the medical device connection station 2 may comprise a docking interface on which the medical device 1 may be positioned. The medical device connection station may include wireless telecommunications transfer electronics and a SIM (Subscriber Identity Module) card for connection to a mobile phone network 3 such as the GSM (Global System for Mobile communication) or the UMTS (Universal Mobile Telecommunications System). When placed on the medical device connection station 2, the medical device 1 communicates with the medical device connection station 2 by infrared, radiofrequency or electrical connection. Via a graphical user interface 4 and buttons 5 on the medical device 1, the patient may transmit the first data and the identification code from the medical device 1 to the medical device connection station 2. The first data and the identification code are then transmitted via the mobile phone network 3 to the distant server system 6.

The server system 6 acquires, stores and processes the first data. The data transmission between the medical device connection station 2 and the server system 6 is encrypted. The data is stored confidentially in the server system 6 and processed so as to be easily and securely accessible to the patient's care giver via a communication network such as the Internet 7. Thus, using a user interface device 18, typically a computer or a digital tablet, and via an encrypted communication, the physician may access the data stored in the server system 6 for his/her patient.

In addition to the aforementioned first data, the server system 6 acquires from the patient second data relating to a physiological state of the patient. These second data are sent to the server system 6 by a smart mobile phone 18c via the mobile phone network 3, by a computer 18a via the Internet network 7 and/or by any other terminal of the patient. The second data may include patient-reported outcomes (PROs), which may include responses to a clinically validated questionnaire previously sent by the server system 6 to the smart mobile phone 18c and/or the computer 18a upon request by the physician. The patient regularly, when prompted by his UID 18, or at will, answers the questionnaire and the answers are sent to the server system 6, which stores and processes them. The questionnaire may contain questions permitting evaluating the level of general health, pain, cognition, fatigue, bladder condition, bowel condition, sexual satisfaction or other health criteria of the patient. The second data may further include health test results. The tests may be done by the patient at home using the computer 18a and test apparatuses such as motion sensors connected to the computer 18a by e.g. a radiofrequency connection (e.g. Bluetooth® or Wi-Fi). The tests may be vision tests, walk tests, cognition tests and/or mobility tests.

The second data may also include measured physiological data, such as the body temperature, the blood pressure or the height (in the case of a growth hormone treatment) of the patient. Like the first data, the second data is transmitted in an encrypted manner and confidentially stored in the server system 6.

When the physician connects his/her terminal 8 to the server system 6, he/she may access the first and second data stored therein for the patient. According to the present invention, and as shown in Figure 6, two graphical representations 11, 12 respectively obtained from the first and second data stored in the server system 6 are used to generate a composite report, whereby first and second data are simultaneously displayed on the screen of the terminal 8 so that they can be viewed together. By the term "simultaneously" it is meant that there exists at least a period of time during which the two graphical representations 11, 12 are displayed together. The two graphical representations 11, 12 may be side-by-side, superimposed or one above the other as illustrated.

The first graphical representation 11 represents the adherence to the medical treatment, in particular the extent to which the patient follows the medical instructions such as dose and injection frequency, as a function of time. Thus, an adherence of 100% indicates that the patient has strictly followed the medical prescription, while an adherence lower than 100% indicates that the patient did not inject all prescribed doses. The first graphical representation 11 may for instance be in the form of a bar graph, as illustrated, and may be combined with a curve 13 representing a condition of the patient, for instance in the case of a neurodegenerative disease such as Multiple Sclerosis, the EDSS (Expanded Disability Status Scale) score of the patient which is provided to the server system 6 by the physician. The second graphical representation 12 may advantageously include one or several curves 14 respectively representing the aforementioned patient-reported outcomes as a function of time. The physician may select which patient-reported outcome(s) to display. Although not shown, the second graphical representation 12 may also include curves or other graphical data representing the health test results and the measured physiological data.

Since the graphical representations 11, 12 are simultaneously displayed on the physician's terminal 18, the physician may easily analyse the data, and may observe the influence of the adherence on the health of the patient. The physician may then evaluate the efficacy of the medical treatment, adjust the prescription and/or incite the patient to more strictly follow the prescription, and/or propose accompanying therapeutic measures.

The adherence may be calculated by the software components in the server system 6 based on the first data provided by the medical device 1 and the medical device connection station 2. The first and second graphical representations 11, 12 are also produced by software components in the server system 6. However, in a variant, a client software application installed on the physician's computer terminal 18 could calculate the adherence and produce the graphical representations 11, 12 based on raw data provided by the server system 6. In another variant, the adherence could be calculated by a software component in the server system 6 and the graphical representations 11, 12 could be produced by a client software application installed on the computer terminal 18. In still another variant, the adherence could be calculated by a software component in the medical device 1.

The first data may be sent by the server system 6 to the mobile phone 18c, the computer 18a and/or any other terminal of the patient upon request by the physician or automatically, thus enabling the patient to monitor the adherence.

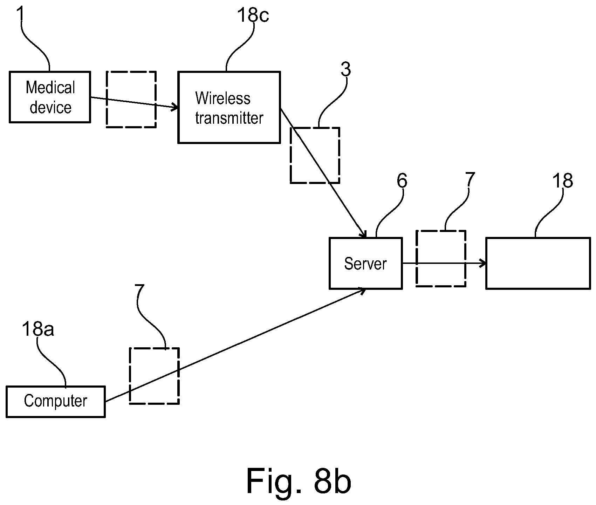

In a variant of the invention, shown in Figures 8b and 7b, the medical device 1 is a fully mechanical injection device such as the Rebidose®, but bears a smart label 19. The smart label 19 typically includes an NFC (Near Field Communication) chip and a QR (Quick Response) code. The identification code of the medical device is stored in the NFC chip and, also, represented by the QR code. If the smart mobile phone 18c is equipped with NFC means, tapping the smart mobile phone 18c to the medical device 1 triggers transmission of the identification code into the smart mobile phone 18c. Otherwise, the identification code may be accessed by the smart mobile phone 18c by reading the QR code. The patient then performs the injection with the medical device 1 and confirms the injection to the smart mobile phone 18c, which can in turn transmit the injection data (the aforementioned "first data") and the identification code to the server system 6. Thus, in this variant, the mobile phone 18c and the transmitter are one and a same device. The data processing which, in the embodiment of Figure 1, was performed by the medical device, is here performed by the smart mobile phone 18c.

A further variant of the invention is shown in Figure 3. Here, the medical device 1 is integrated with the first data input device and the second data input device. The medical device 1 acquires and stores the first and second data. The medical device docks to a wireless medical device connection station 2 and communicates the data to the medical device connection station via infrared. The medical device connection station 2 transmits the data to the server system 6 via a mobile network 3. A computer 18a belonging to the patient acquires second data, relating to a physiological state of the patient, and transmits it to the server system 6 via the internet 7. The server system 6 stores and processes the first and second data and transmits it to the care giver's computer 18 via the internet 7.

The present invention may also apply to other medical devices than those shown in Figures 7a, 7b, for example to electronic pill dispensers or pill dispensers having smart blisters. The patient may in fact need to use different medical devices depending on the medical treatments he/she follows. Since each medical device has a unique identification code, the server system 6 knows which medical device the acquired first data is concerned with.

The server system 6 is not necessarily located at one place but may be comprised of several distant servers communicating with each other through telecommunication networks. For example, the server system 6 may be comprised of a first server which stores and processes the data and a web server which makes the data available to the physicians through the Internet.

The computerized patient care system according to the invention may be referred to as an eHealth medical platform. Advantageously, the platform is secure and modular. The platform provides a set of applications to share information among care givers and patients in order to monitor and act upon a patient's condition, adherence to a dosage regimen and progress of a disease.

The invention enables a care giver or a patient to monitor the adherence to a medical treatment regimen and to monitor the efficacy of a medical treatment regimen.

The patient care system according to the invention has numerous benefits, including :
- the ability to inform the patient if their adherence is low and if they are not uploading often enough provision of coaching aides, or to provide advance warning such as telling the patient to take cognition training, rest or take a cold shower if their patient reported outcomes (PROs) show that they might relapse, and therefore help to prevent relapse by improving adherence and providing coaching
- patient support groups and field nurses will have access to the patients history of adherence and PROs they will be able to provide more tailored coaching to the patient
- the physician is better prepared for the patent's visit
- if adherence is low, the physician can link low adherence to side effects, which can then be better managed
- adherence in many diseases, in particular multiple sclerosis, has never been correlated to PROs so the system will help us to learn more about the disease , for instance the conditions leading to relapse and how it can be avoided.

## Claims

1. Patient care system comprising a medical device (1) for administering a medical treatment to a patient (15a) and a server system (6) configured to receive and transmit data via a communications network (16) to and from users including patients (15a) and health care professionals (15b), the server system further configured to process and store data related to patient care, wherein the server system comprises a database (6c) configured to store data related to patient care, an application server (6b) including patient care software components for disease management (36) and patient information management program (32), a communication server (6a) including a web server software application for data transfer through the internet, the patient care software components configured to receive medical device usage data comprising data on the usage of said medical device transferred through the communications network and to receive second data, i.e. patient data (32c), from one or more second data input devices, and further configured to process said medical device usage data in conjunction with patient data (32c), selected from a clinically validated questionnaire, health test results and physiological data to generate a report (32f) or a plurality of reports related to the treatment of the patient, the reports being accessible remotely via the communications network by registered users of the patient care system as a function of respective roles and privileges of the registered user stored in the server system, the patient information management program comprising a reports component configured to generate reports, in the form of tables, charts, lists, diagrams or graphical representations based on information selected from drug administration history, adherence data, patient outcome reports, patient health reports, patient physiological data reports, medical device settings, treatment regimen data, and any combination of aforesaid information, the reports component being configured to form composite reports for simultaneous display on a user interface device (UID) display, including composite adherence and patient outcome reports to facilitate evaluation of the effects of non-adherence to the treatment regimen or the efficacy of the medical treatment, and wherein the system is operable to time stamp the medical device usage data, first data, such that the time and/or date of use of the medical device is recorded; and wherein the system is operable to time stamp the patient data, second data, such that the time and/or date of a physiological state of a patient is recorded, and wherein the composite adherence report is represented by a first graphical representation, and the patient outcome report relates to a physiological state of the patient and is represented by a second graphical representation, the first and second representations having a common time scale, and such that the composite adherence report and the patient outcome report are displayed for a common time interval, such that an operator can compare and/or correlate the adherence the treatment regimen to the patient outcome report over a period of time.

2. Patient care system according to claim 1, wherein the communication server further comprises a remote service data upload software application (26) configured for wireless telecommunication technology (WTT) data transfer.

3. Patient care system according to claim 2, further comprising a medical device connection station (22) comprising a wireless telecommunication technology (WTT) transmitter configured for connection to the server system via a wireless telecommunications network, the medical device connection station configured to interconnect to the medical device and to directly upload medical device usage data to the patient information management program application via the WTT remote service data upload application on the server system.

4. Patient care system according to any preceding claim further comprising a client side software application installable on a mobile user interface device (UID) (18b, 18c), such as a phone or computer tablet, configured to upload medical device usage data to the patient information management program application via the WTT remote service data upload application on the server system.

5. Patient care system according to claim 1, wherein the medical device incorporates a wireless telecommunication technology (WTT) transmitter configured for connection to the server system via a wireless telecommunications network, the medical device configured to upload medical device usage data to the patient information management program application via a WTT remote service data upload application (26) on the server system.

6. Patient care system according to claim 1, wherein the composite adherence report is displayed on a first time scale and the patient outcome report is displayed on a second time scale, wherein the first and second time scales are aligned and simultaneously displayed such that the composite adherence report and the patient outcome report are displayed for a common time interval, such that an operator can compare and/or correlate the adherence the treatment regimen to the patient outcome report over a period of time.

7. Patient care system according to claim 1, wherein the composite adherence report is displayed on a first time scale and at least one patient outcome report is displayed on a second time scale, wherein the first and second time scales are displaced in relation to each other, such that the patient outcome report is compensated for a time delay in noticeable/measurable patient outcome, such that an operator can compare and/or correlate the adherence to the patient outcome report over a period of time.

8. Patient care system according to any one of claims 6-7, wherein the time scale displays adherence data and patient outcome reports per hour, day, week, month, year or any combination thereof

9. Patient care system according to any preceding claim, wherein the server system further comprises a notification services software component (30b) configured to transmit email and/or SMS (Short Message Service) notifications to patients and optionally other users of the system, notifications selected from a group including alarms, visit reminders, treatment regime information, health information, drug information.

10. Patient care system according to any preceding claim, wherein the server system further comprises patient therapy services software components including tests (40a) accessible online by patients, the software component configured to capture results of tests, such as vision and walking tests, and feed them into the reports software component.

11. Patient care system according to any preceding claim, wherein the server system further comprises patient therapy services software components including training exercises (40b) accessible online by patients, including training exercises selected from cognition training and mobility training.

12. Patient care system according to either of the directly two preceding claims, patient therapy services may further be configured to receive data from sensors relating to physiological measurements of the patient's measured physiological data captured automatically by sensors and transmitted to the server system via the patient's user interface device (18) and/or medical device (1) and/or by the sensing or training device, physiological data selected from body temperature, blood pressure, pulse rate, galvanic skin response, surface electromyography, electroencephalography measurements, oculography measurements, electrocardiography measurements, breathing sensor measurements, blood sugar sensor measurements.

13. Patient care system according to claim 1, wherein the patient care system is configured for a patient with multiple sclerosis.

14. Patient care system according to claim 1, wherein the patient care system is configured for a patient with growth hormone deficiency.

15. Patient care system according to claim 1, wherein the patient care system is configured for a patient with rheumatoid arthritis, or for a patient with juvenile rheumatoid arthritis, or for a patient with psoriasis, or for a patient with plaque psoriasis, or for a patient with Crohn's disease, or for a patient with juvenile Crohn's disease, or for a patient with asthma, or for a patient with psoriatic arthritis, or for a patient with ulcerative colitis, or for a patient with systemic lupus erythematosus, or for a patient with ankylosing spondylitis.

## Patentansprüche

1. Patientenvorsorgesystem, das eine medizinische Vorrichtung (1) zum Verwalten einer medizinischen Behandlung eines Patienten (15a) und ein Serversystem (6) umfasst, das eingerichtet ist, Daten über ein Kommunikationsnetzwerk (16) zu und von Nutzern zu empfangen und zu übertragen, die Patienten (15a) und Gesundheitsvorsorgefachleute (15b) umfassen, wobei das Serversystem ferner eingerichtet ist, Daten zu verarbeiten und zu speichern, die sich auf die Patientenvorsorge beziehen, wobei das Serversystem eine Datenbank (6c) umfasst, die eingerichtet ist, Daten zu speichern, die sich auf die Patientenvorsorge beziehen, einen Anwendungsserver (6b), der Patientenvorsorge-Softwarekomponenten zur Krankheitsverwaltung (36) und ein Patienteninformations-Verwaltungsprogramm (32) umfasst, einen Kommunikationsserver (6a), der eine Webserver-Softwareanwendung für Datenübertragung über das Internet umfasst, wobei die Patientenvorsorge-Softwarekomponenten eingerichtet sind, medizinische Vorrichtungs-Nutzungsdaten zu empfangen, die Daten über die Nutzung der medizinischen Vorrichtung umfassen, die über das Kommunikationsnetzwerk übertragen werden, und zweite Daten zu empfangen, d.h. Patientendaten (32c) von einem oder mehreren zweiten Dateneingabevorrichtungen, und ferner eingerichtet, die medizinischen Vorrichtungs-Nutzungsdaten in Verbindung mit Patientendaten (32c) zu verarbeiten, die aus einem klinisch validierten Fragebogen, Gesundheitstestergebnissen und physiologischen Daten ausgewählt werden, um einen Bericht (32f) oder eine Vielzahl von Berichten zu erstellen, die sich auf die Behandlung des Patienten beziehen, wobei die Berichte über das Kommunikationsnetzwerk von registrierten Nutzern des Patientenvorsorgesystems in Abhängigkeit von den jeweiligen Rollen und Privilegien des registrierten Nutzers, die im Serversystem gespeichert sind, aus der Ferne zugänglich sind, wobei das Patienteninformations-Verwaltungsprogramm eine Berichtskomponente umfasst, die eingerichtet ist, Berichte in Form von Tabellen, Graphiken, Listen, Diagrammen oder grafischen Darstellungen basierend auf Informationen zu erstellen, die aus einer Arzneimittelverwaltungshistorie, Adhärenzdaten, Patientenergebnisberichten, Patientengesundheitsberichten, Berichten über physiologische Daten des Patienten, medizinischen Vorrichtungs-Einstellungen, Behandlungsregimedaten und einer beliebigen Kombination der oben genannten Informationen ausgewählt werden, wobei die Berichtskomponente eingerichtet ist, zusammengestellte Berichte für die gleichzeitige Anzeige auf einer Nutzerschnittstellenvorrichtungs (UID)-Anzeige zu erstellen, die zusammengestellte Adhärenz- und Patientenergebnisberichte umfassen, um die Auswertung der Effekte einer Nicht-Adhärenz des Behandlungsregimes oder der Wirksamkeit der medizinischen Behandlung zu erleichtern, und wobei das System so betrieben werden kann, dass die medizinischen Vorrichtungs-Nutzungsdaten, erste Daten, mit einem Zeitstempel versehen werden, so dass die Zeit und/oder das Datum der Nutzung der medizinischen Vorrichtung aufgezeichnet werden; und wobei das System betrieben werden kann, die Patientendaten, zweite Daten, mit einem Zeitstempel zu versehen, so dass die Zeit und/oder das Datum eines physiologischen Zustands eines Patienten aufgezeichnet werden, und wobei der zusammengestellte Adhärenzbericht durch eine erste grafische Darstellung dargestellt wird und der Patientenergebnisbericht sich auf einen physiologischen Zustand des Patienten bezieht und durch eine zweite grafische Darstellung dargestellt wird, wobei die erste und zweite Darstellungen eine gemeinsame Zeitskala haben und so, dass der zusammengestellte Adhärenzbericht und der Patientenergebnisbericht für ein gemeinsames Zeitintervall angezeigt werden, so dass ein Bediener die Adhärenz des Behandlungsregimes mit dem Patientenergebnisbericht über einen bestimmten Zeitraum vergleichen und/oder korrelieren kann.

2. Patientenvorsorgesystem nach Anspruch 1, wobei der Kommunikationsserver ferner eine Ferndienst-Datenupload-Softwareanwendung (26) umfasst, die für die drahtlose Telekommunikationstechnologie (WTT)-Datenübertragung eingerichtet ist.

3. Patientenvorsorgesystem nach Anspruch 2, das ferner eine medizinische Vorrichtungs-Verbindungsstation (22) umfasst, die einen drahtlosen Telekommunikationstechnologie (WTT)-Sender umfasst, der für die Verbindung zum Serversystem über ein drahtloses Telekommunikationsnetzwerk eingerichtet ist, wobei die medizinische Vorrichtungs-Verbindungsstation eingerichtet ist, sich mit der medizinischen Vorrichtung zu verbinden und die medizinischen Vorrichtungs-Nutzungsdaten über die WTT-Ferndienst-Datenupload-Anwendung auf dem Serversystem direkt in die Patienteninformations-Verwaltungsprogramm-Anwendung hochzuladen.

4. Patientenvorsorgesystem nach einem vorhergehenden Anspruch, das ferner eine clientseitige Softwareanwendung umfasst, die auf einer mobilen Nutzerschnittstellenvorrichtung (UID) (18b, 18c) installiert werden kann, wie z.B. einem Telefon oder einem Computertablett, das eingerichtet ist, medizinische Vorrichtungs-Nutzungsdaten über die WTT-Ferndienst-Datenupload-Anwendung auf dem Serversystem in die Patienteninformations-Verwaltungsprogramm-Anwendung hochzuladen.

5. Patientenvorsorgesystem nach Anspruch 1, wobei die medizinische Vorrichtung einen drahtlosen Telekommunikationstechnologie (WTT)-Sender umfasst, der für die Verbindung zum Serversystem über ein drahtloses Telekommunikationsnetzwerk eingerichtet ist, wobei die medizinische Vorrichtung eingerichtet ist, medizinische Vorrichtungs-Nutzungsdaten über eine WTT-Ferndienst-Datenupload-Anwendung (26) auf dem Serversystem in die Patienteninformations-Verwaltungsprogramm-Anwendung hochzuladen.

6. Patientenvorsorgesystem nach Anspruch 1, wobei der zusammengestellte Adhärenzbericht auf einer ersten Zeitskala und der Patientenergebnisbericht auf einer zweiten Zeitskala angezeigt wird, wobei die erste und die zweite Zeitskalen so ausgerichtet und gleichzeitig angezeigt werden, dass der zusammengestellte Adhärenzbericht und der Patientenergebnisbericht für ein gemeinsames Zeitintervall angezeigt werden, so dass ein Bediener die Adhärenz des Behandlungsregimes mit dem Patientenergebnisbericht über einen Zeitraum vergleichen und/oder korrelieren kann.

7. Patientenvorsorgesystem nach Anspruch 1, wobei der zusammengestellte Adhärenzbericht auf einer ersten Zeitskala und mindestens ein Patientenergebnisbericht auf einer zweiten Zeitskala angezeigt wird, wobei die erste und zweite Zeitskalen in Bezug zueinander verschoben sind, so dass der Patientenergebnisbericht für eine Zeitverzögerung im wahrnehmbaren/messbaren Patientenergebnis kompensiert wird, so dass ein Bediener die Adhärenz mit dem Patientenergebnisbericht über einen Zeitraum vergleichen und/oder korrelieren kann.

8. Patientenvorsorgesystem nach einem der Ansprüche 6-7, wobei die Zeitskala die Adhärenzdaten und Patientenergebnisberichte pro Stunde, Tag, Woche, Monat, Jahr oder einer beliebigen Kombination davon anzeigt.

9. Patientenvorsorgesystem nach einem vorhergehenden Anspruch, wobei das Serversystem ferner eine Benachrichtigungsdienst-Softwarekomponente (30b) umfasst, die eingerichtet ist, E-Mail- und/oder Kurzmitteilungsdienst (SMS)-Benachrichtigungen an Patienten und optional an andere Nutzer des Systems zu übertragen, wobei die Benachrichtigungen aus einer Gruppe ausgewählt werden, die Alarme, Besuchserinnerungen, Behandlungsregimeinformationen, Gesundheitsinformationen und Arzneimittelinformationen umfasst.

10. Patientenvorsorgesystem nach einem vorhergehenden Anspruch, wobei das Serversystem ferner Patiententherapiedienste-Softwarekomponenten umfasst, die Tests (40a) umfassen, die den Patienten online zugänglich sind, wobei die Softwarekomponente eingerichtet ist, Ergebnisse von Tests zu erfassen, wie z.B. Seh- und Gehtests, und sie in die Berichte-Softwarekomponente einzuspeisen.

11. Patientenvorsorgesystem nach einem vorhergehenden Anspruch, wobei das Serversystem ferner Patiententherapiedienste-Softwarekomponenten umfasst, die Trainingsübungen (40b) umfassen, die den Patienten online zugänglich sind, die Trainingsübungen umfassen, die aus Kognitionstraining und Mobilitätstraining ausgewählt werden.

12. Patientenvorsorgesystem nach einem der beiden unmittelbar vorhergehenden Ansprüche, wobei die Patiententherapiedienste ferner eingerichtet sein können, Daten von Sensoren zu empfangen, die sich auf physiologische Messungen der gemessenen physiologischen Daten des Patienten beziehen, die automatisch von Sensoren erfasst und an das Serversystem über die Nutzerschnittstellenvorrichtung des Patienten (18) und/oder die medizinische Vorrichtung (1) und/oder durch die Erfassungs- oder Trainingsvorrichtung übertragen werden, wobei die physiologischen Daten aus Körpertemperatur, Blutdruck, Pulsfrequenz, galvanischer Hautreaktion, Oberflächen-Elektromyographie, Elektroenzephalographie-Messungen, Okulographie-Messungen, Elektrokardiographie-Messungen, Atemsensor-Messungen, Blutzuckersensor-Messungen ausgewählt werden.

13. Patientenvorsorgesystem nach Anspruch 1, wobei das Patientenvorsorgesystem für einen Patienten mit Multipler Sklerose eingerichtet ist.

14. Patientenvorsorgesystem nach Anspruch 1, wobei das Patientenvorsorgesystem für einen Patienten mit Wachstumshormonmangel eingerichtet ist.

15. Patientenvorsorgesystem nach Anspruch 1, wobei das Patientenvorsorgesystem für einen Patienten mit rheumatoider Arthritis eingerichtet ist oder für einen Patienten mit juveniler rheumatoider Arthritis oder für einen Patienten mit Psoriasis oder für einen Patienten mit Plaque-Psoriasis oder für einen Patienten mit Morbus Crohn, oder für einen Patienten mit juvenilem Morbus Crohn, oder für einen Patienten mit Asthma, oder für einen Patienten mit psoriatischer Arthritis, oder für einen Patienten mit Colitis ulcerosa, oder für einen Patienten mit systemischem Lupus erythematodes, oder für einen Patienten mit Spondylitis ankylosans.

## Revendications

1. Système de soins de patient comprenant un dispositif médical (1) servant à administrer un traitement médical à un patient (15a) et un système serveur (6) configuré pour recevoir et émettre des données par l'intermédiaire d'un réseau de communication (16) en provenance et à destination d'utilisateurs y compris des patients (15a) et des professionnels des soins de santé (15b), le système serveur étant en outre configuré pour traiter et stocker des données relatives à des soins de patient, **dans lequel** le système serveur comprend une base de données (6c) configurée pour stocker des données relatives à des soins de patient, un serveur d'applications (6b) comportant des composants logiciels de soins de patient pour la prise en charge de la maladie (36) et un programme de gestion d'informations de patient (32), un serveur de communication (6a) comportant une application logicielle de serveur web pour le transfert de données par l'internet, les composants logiciels de soins de patient étant configurés pour recevoir des données d'usage de dispositif médical comprenant des données sur l'usage dudit dispositif médical transférées par le réseau de communication et pour recevoir des secondes données, à savoir des données de patient (32c), en provenance d'un ou de plusieurs dispositifs d'entrée de secondes données, et étant en outre configurés pour traiter lesdites données d'usage de dispositif médical conjointement avec des données de patient (32c), sélectionnées parmi un questionnaire validé cliniquement, des résultats de test de santé et des données physiologiques, afin de générer un compte rendu (32f) ou une pluralité de comptes rendus relatifs au traitement du patient, les comptes rendus étant accessibles à distance par l'intermédiaire du réseau de communication à des utilisateurs enregistrés du système de soins de patient en fonction de rôles et de privilèges respectifs de l'utilisateur enregistré stockés dans le système serveur, le programme de gestion d'informations de patient comprenant un composant de comptes rendus configuré pour générer des comptes rendus, sous la forme de tableaux, de cartes, de listes, de diagrammes ou de représentations graphiques, sur la base d'informations sélectionnées parmi un historique d'administration de médicaments, des données d'observance, des comptes rendus d'évolution de l'état de santé, des comptes rendus de santé de patient, des comptes rendus de données physiologiques de patient, des réglages de dispositif médical, des données de régime de traitement, et toute combinaison des informations susmentionnées, le composant de comptes rendus étant configuré pour former des comptes rendus composites destinés à être affichés simultanément sur un écran de dispositif d'interface utilisateur (UID), y compris des comptes rendus d'observance et d'évolution de l'état de santé composites pour faciliter l'évaluation des effets de non observance du régime de traitement ou de l'efficacité du traitement médical, et le système étant utilisable pour horodater les données d'usage de dispositif médical, premières données, de manière que l'heure et/ou la date d'utilisation du dispositif médical soient enregistrées ; et le système étant utilisable pour horodater les données de patient, secondes données, de manière que l'heure et/ou la date d'un état physiologique d'un patient soient enregistrées, et le compte rendu d'observance composite étant représenté par une première représentation graphique, et le compte rendu d'évolution de l'état de santé étant relatif à un état physiologique du patient et étant représenté par une seconde représentation graphique, les première et seconde représentations ayant une échelle de temps commune, et de manière que le compte rendu d'observance composite et le compte rendu d'évolution de l'état de santé soient affichés pour un intervalle de temps commun, de manière qu'un opérateur puisse comparer et/ou corréler l'observance du régime de traitement à l'évolution de l'état de santé sur une certaine période de temps.

2. Système de soins de patient selon la revendication 1, dans lequel le serveur de communication comprend en outre une application logicielle de téléversement de données de service à distance (26) configurée pour le transfert de données par technologie de télécommunication sans fil (WTT).

3. Système de soins de patient selon la revendication 2, comprenant en outre une station de connexion de dispositif médical (22) comprenant un émetteur de technologie de télécommunication sans fil (WTT) configuré en vue d'une connexion au système serveur par l'intermédiaire d'un réseau de télécommunications sans fil, la station de connexion de dispositif médical étant configurée pour s'interconnecter au dispositif médical et pour téléverser directement des données d'usage de dispositif médical vers l'application programme de gestion d'informations de patient par l'intermédiaire de l'application de téléversement de données de service à distance WTT sur le système serveur.

4. Système de soins de patient selon l'une quelconque des revendications précédentes, comprenant en outre une application logicielle côté client installable sur un dispositif d'interface utilisateur (UID) mobile (18b, 18c), tel qu'un téléphone ou une tablette électronique, configuré pour téléverser des données d'usage de dispositif médical vers l'application programme de gestion d'informations de patient par l'intermédiaire de l'application de téléversement de données de service à distance WTT sur le système serveur.

5. Système de soins de patient selon la revendication 1, dans lequel le dispositif médical incorpore un émetteur de technologie de télécommunication sans fil (WTT) configuré en vue d'une connexion au système serveur par l'intermédiaire d'un réseau de télécommunications sans fil, le dispositif médical étant configuré pour téléverser des données d'usage de dispositif médical vers l'application programme de gestion d'informations de patient par l'intermédiaire d'une application de téléversement de données de service à distance WTT (26) sur le système serveur.

6. Système de soins de patient selon la revendication 1, dans lequel le compte rendu d'observance composite est affiché sur une première échelle de temps et le compte rendu d'évolution de l'état de santé est affiché sur une seconde échelle de temps, les première et seconde échelles de temps étant alignées et affichées simultanément de manière que le compte rendu d'observance composite et le compte rendu d'évolution de l'état de santé soient affichés pour un intervalle de temps commun, de manière qu'un opérateur puisse comparer et/ou corréler l'observance du régime de traitement au compte rendu d'évolution de l'état de santé sur une certaine période de temps.

7. Système de soins de patient selon la revendication 1, dans lequel le compte rendu d'observance composite est affiché sur une première échelle de temps et au moins un compte rendu d'évolution de l'état de santé est affiché sur une seconde échelle de temps, les première et seconde échelles de temps étant décalées l'une par rapport à l'autre, de manière que le compte rendu d'évolution de l'état de santé soit compensé d'un retard temporel dans une évolution de l'état de santé perceptible/mesurable, de manière qu'un opérateur puissance comparer et/ou corréler l'observance au compte rendu d'évolution de l'état de santé sur une certaine période de temps.

8. Système de soins de patient selon l'une quelconque des revendications 6 à 7, dans lequel l'échelle de temps affiche des données d'observance et des comptes rendus d'évolution de l'état de santé par heure, jour, semaine, mois, année, ou toute combinaison de ces unités.

9. Système de soins de patient selon l'une quelconque des revendications précédentes, dans lequel le système serveur comprend en outre un composant logiciel de services de notification (30b) configuré pour transmettre des notifications par courriel et/ou SMS (service de messages courts) à des patients et facultativement à d'autres utilisateurs du système, les notifications étant sélectionnées dans un groupe comprenant alarmes, rappels de consultation, informations de régime de traitement, informations de santé, informations de médicaments.

10. Système de soins de patient selon l'une quelconque des revendications précédentes, dans lequel le système serveur comprend en outre des composants logiciels de services de thérapie de patient comportant des tests (40a) accessibles en ligne à des patients, le composant logiciel étant configuré pour capturer des résultats de tests, tels que des tests de vision et de marche, et les introduire dans le composant logiciel de comptes rendus.

11. Système de soins de patient selon l'une quelconque des revendications précédentes, dans lequel le système serveur comprend en outre des composants logiciels de services de thérapie de patient comportant des exercices d'entraînement (40b) accessibles en ligne à des patients, y compris des exercices d'entraînement sélectionnés parmi entraînement cognitif et entraînement à la mobilité.

12. Système de soins de patient selon l'une ou l'autre des deux revendications directement précédentes, les services de thérapie de patient peuvent en outre être configurés pour recevoir des données provenant de capteurs relatives à des mesures physiologiques des données physiologiques mesurées du patient capturées automatiquement par des capteurs et transmises au système serveur par l'intermédiaire du dispositif d'interface utilisateur (18) et/ou du dispositif médical (1) du patient et/ou par le dispositif de détection ou d'entraînement, les données physiologiques étant sélectionnées parmi température corporelle, pression sanguine, fréquence du pouls, réflexe psychogalvanique, électromyogramme de surface, mesures d'électroencéphalographie, mesures d'oculographie, mesures d'électrocardiographie, mesures de capteur de respiration, mesures de capteur de glycémie.

13. Système de soins de patient selon la revendication 1, le système de soins de patient étant configuré pour un patient souffrant de sclérose en plaques.

14. Système de soins de patient selon la revendication 1, le système de soins de patient étant configuré pour un patient souffrant de déficit en hormone de croissance.

15. Système de soins de patient selon la revendication 1, le système de soins de patient étant configuré pour un patient souffrant de polyarthrite rhumatoïde, ou pour un patient souffrant de polyarthrite juvénile, ou pour un patient souffrant de psoriasis, ou pour un patient souffrant de psoriasis en plaques, ou pour un patient souffrant de maladie de Crohn, ou pour un patient souffrant de maladie de Crohn juvénile, ou pour un patient souffrant d'asthme, ou pour un patient souffrant d'arthrite psoriasique, ou pour un patient souffrant de rectocolite hémorragique, ou pour un patient souffrant de lupus érythémateux disséminé, ou pour un patient souffrant de spondylarthrite ankylosante.
